# EUROPEAN PATENT SPECIFICATION

(11) **EP 2 598 177 B1**
(45) Date of publication and mention of the grant of the patent: **17.06.2015**
(21) Application number: 11736402.6
(22) Date of filing: 29.07.2011
(51) Int. Cl.: A61K 38/18, A61L 15/44, A61L 27/54, A61L 31/16, A61L 26/00

(54) **DRUG DELIVERY DEVICES AND GROWTH FACTOR FORMULATIONS FOR ACCELERATED WOUND HEALING**
WIRKSTOFFFREISETZUNGSVORRICHTUNGEN UND WACHSTUMSFAKTORFORMULIERUNGEN FÜR BESCHLEUNIGTE WUNDHEILUNG
DISPOSITIFS D'ADMINISTRATION DE MÉDICAMENTS ET FORMULATIONS DE FACTEUR DE CROISSANCE POUR ACCÉLÉRER LA CICATRISATION DE PLAIES

(30) Priority: 30.07.2010 EP 10171488
(43) Date of publication of application: 05.06.2013
(73) Proprietor: Biopharm Gesellschaft Zur Biotechnologischen Entwicklung Von Pharmaka mbH, 69115 Heidelberg (DE)
(72) Inventor: PLÖGER, Frank, 69245 Bammental (DE)
(74) Representative: Weickmann & Weickmann
(86) International application number: PCT/EP2011/063128
(87) International publication number: WO 2012/013790

(56) References cited:
- EP-A1- 1 698 691
- WO-A1-2008/143867
- FR-A1- 2 919 188
- US-B2- 6 669 980
- NISHANT KUMAR JAIN ET AL: "Effect of trehalose on protein structure", PROTEIN SCIENCE, vol. 18, no. 1, 1 January 2009 (2009-01-01), pages NA-NA, XP55011499, ISSN: 0961-8368, DOI: 10.1002/pro.3

### SEQUENCE LISTING

<110> Biopharm Gesellschaft zur biotechnologischen Entwicklung von Pharmaka mbH
<120> Drug delivery devices and growth factor formulations for accelerated wound healing
<130> 48756P EP
<160> 4
<170> PatentIn version 3.3
<210> 1
   <211> 2703
   <212> DNA
   <213> homo sapiens
<220>
   <221> CDS
   <222> (640)..(2142)
   <223> GDF-5 precursor
<400> 1
   ccatggcctc gaaagggcag cggtgatttt tttcacataa atatatcgca cttaaatgag     60
   tttagacagc atgacatcag agagtaatta aattggtttg ggttggaatt ccgtttccaa    120
   ttcctgagtt caggtttgta aaagattttt ctgagcacct gcaggcctgt gagtgtgtgt    180
   gtgtgtgtgt gtgtgtgtgt gtgtgtgtga agtattttca ctggaaagga ttcaaaacta    240
   gggggaaaaa aaaactggag cacacaggca gcattacgcc attcttcctt cttggaaaaa    300
   tccctcagcc ttatacaagc ctccttcaag ccctcagtca gttgtgcagg agaaaggggg    360
   cggttggctt tctcctttca agaacgagtt attttcagct gctgactgga gacggtgcac    420
   gtctggatac gagagcattt ccactatggg actggataca aacacacacc cggcagactt    480
   caagagtctc agactgagga gaaagccttt ccttctgctg ctactgctgc tgccgctgct    540
   tttgaaagtc cactcctttc atggtttttc ctgccaaacc agaggcacct ttgctgctgc    600
   cgctgttctc tttggtgtca ttcagcggct ggccagagg atg aga ctc ccc aaa       654
                                             Met Arg Leu Pro Lys
                                             1               5
   ctc ctc act ttc ttg ctt tgg tac ctg gct tgg ctg gac ctg gaa ttc      702
   Leu Leu Thr Phe Leu Leu Trp Tyr Leu Ala Trp Leu Asp Leu Glu Phe
                   10                  15                  20
   atc tgc act gtg ttg ggt gcc cct gac ttg ggc cag aga ccc cag ggg      750
   Ile Cys Thr Val Leu Gly Ala Pro Asp Leu Gly Gln Arg Pro Gln Gly
               25                  30                  35
   acc agg cca gga ttg gcc aaa gca gag gcc aag gag agg ccc ccc ctg      798
   Thr Arg Pro Gly Leu Ala Lys Ala Glu Ala Lys Glu Arg Pro Pro Leu
           40                  45                  50
   gcc cgg aac gtc ttc agg cca ggg ggt cac agc tat ggt ggg ggg gcc      846
   Ala Arg Asn Val Phe Arg Pro Gly Gly His Ser Tyr Gly Gly Gly Ala
       55                  60                  65
   acc aat gcc aat gcc agg gca aag gga ggc acc ggg cag aca gga ggc      894
   Thr Asn Ala Asn Ala Arg Ala Lys Gly Gly Thr Gly Gln Thr Gly Gly
   70                  75                  80                  85
   ctg aca cag ccc aag aag gat gaa ccc aaa aag ctg ccc ccc aga ccg      942
   Leu Thr Gln Pro Lys Lys Asp Glu Pro Lys Lys Leu Pro Pro Arg Pro
                   90                  95                  100
   ggc ggc cct gaa ccc aag cca gga cac cct ccc caa aca agg cag gct      990
   Gly Gly Pro Glu Pro Lys Pro Gly His Pro Pro Gln Thr Arg Gln Ala
               105                 110                 115
   aca gcc cgg act gtg acc cca aaa gga cag ctt ccc gga ggc aag gca     1038
   Thr Ala Arg Thr Val Thr Pro Lys Gly Gln Leu Pro Gly Gly Lys Ala
           120                 125                 130
   ccc cca aaa gca gga tct gtc ccc agc tcc ttc ctg ctg aag aag gcc     1086
   Pro Pro Lys Ala Gly Ser Val Pro Ser Ser Phe Leu Leu Lys Lys Ala
       135                 140                 145
   agg gag ccc ggg ccc cca cga gag ccc aag gag ccg ttt cgc cca ccc     1134
   Arg Glu Pro Gly Pro Pro Arg Glu Pro Lys Glu Pro Phe Arg Pro Pro
   150                 155                 160                 165
   ccc atc aca ccc cac gag tac atg ctc tcg ctg tac agg acg ctg tcc     1182
   Pro Ile Thr Pro His Glu Tyr Met Leu Ser Leu Tyr Arg Thr Leu Ser
                   170                 175                 180
   gat gct gac aga aag gga ggc aac agc agc gtg aag ttg gag gct ggc     1230
   Asp Ala Asp Arg Lys Gly Gly Asn Ser Ser Val Lys Leu Glu Ala Gly
               185                 190                 195
   ctg gcc aac acc atc acc agc ttt att gac aaa ggg caa gat gac cga     1278
   Leu Ala Asn Thr Ile Thr Ser Phe Ile Asp Lys Gly Gln Asp Asp Arg
           200                 205                 210
   ggt ccc gtg gtc agg aag cag agg tac gtg ttt gac att agt gcc ctg     1326
   Gly Pro Val Val Arg Lys Gln Arg Tyr Val Phe Asp Ile Ser Ala Leu
       215                 220                 225
   gag aag gat ggg ctg ctg ggg gcc gag ctg cgg atc ttg cgg aag aag     1374
   Glu Lys Asp Gly Leu Leu Gly Ala Glu Leu Arg Ile Leu Arg Lys Lys
   230                 235                 240                 245
   ccc tcg gac acg gcc aag cca gcg gcc ccc gga ggc ggg cgg gct gcc     1422
   Pro Ser Asp Thr Ala Lys Pro Ala Ala Pro Gly Gly Gly Arg Ala Ala
                   250                 255                 260
   cag ctg aag ctg tcc agc tgc ccc agc ggc cgg cag ccg gcc tcc ttg     1470
   Gln Leu Lys Leu Ser Ser Cys Pro Ser Gly Arg Gln Pro Ala Ser Leu
               265                 270                 275
   ctg gat gtg cgc tcc gtg cca ggc ctg gac gga tct ggc tgg gag gtg     1518
   Leu Asp Val Arg Ser Val Pro Gly Leu Asp Gly Ser Gly Trp Glu Val
           280                 285                 290
   ttc gac atc tgg aag ctc ttc cga aac ttt aag aac tcg gcc cag ctg     1566
   Phe Asp Ile Trp Lys Leu Phe Arg Asn Phe Lys Asn Ser Ala Gln Leu
       295                 300                 305
   tgc ctg gag ctg gag gcc tgg gaa cgg ggc agg gcc gtg gac ctc cgt     1614
   Cys Leu Glu Leu Glu Ala Trp Glu Arg Gly Arg Ala Val Asp Leu Arg
   310                 315                 320                 325
   ggc ctg ggc ttc gac cgc gcc gcc cgg cag gtc cac gag aag gcc ctg      1662
   Gly Leu Gly Phe Asp Arg Ala Ala Arg Gln Val His Glu Lys Ala Leu
                   330                 335                 340
   ttc ctg gtg ttt ggc cgc acc aag aaa cgg gac ctg ttc ttt aat gag      1710
   Phe Leu Val Phe Gly Arg Thr Lys Lys Arg Asp Leu Phe Phe Asn Glu
               345                 350                 355
   att aag gcc cgc tct ggc cag gac gat aag acc gtg tat gag tac ctg      1758
   Ile Lys Ala Arg Ser Gly Gln Asp Asp Lys Thr Val Tyr Glu Tyr Leu
           360                 365                 370
   ttc agc cag cgg cga aaa cgg cgg gcc cca ctg gcc act cgc cag ggc      1806
   Phe Ser Gln Arg Arg Lys Arg Arg Ala Pro Leu Ala Thr Arg Gln Gly
       375                 380                 385
   aag cga ccc agc aag aac ctt aag gct cgc tgc agt cgg aag gca ctg      1854
   Lys Arg Pro Ser Lys Asn Leu Lys Ala Arg Cys Ser Arg Lys Ala Leu
   390                 395                 400                 405
   cat gtc aac ttc aag gac atg ggc tgg gac gac tgg atc atc gca ccc      1902
   His Val Asn Phe Lys Asp Met Gly Trp Asp Asp Trp Ile Ile Ala Pro
                   410                 415                 420
   ctt gag tac gag gct ttc cac tgc gag ggg ctg tgc gag ttc cca ttg      1950
   Leu Glu Tyr Glu Ala Phe His Cys Glu Gly Leu Cys Glu Phe Pro Leu
               425                 430                 435
   cgc tcc cac ctg gag ccc acg aat cat gca gtc atc cag acc ctg atg      1998
   Arg Ser His Leu Glu Pro Thr Asn His Ala Val Ile Gln Thr Leu Met
           440                 445                 450
   aac tcc atg gac ccc gag tcc aca cca ccc acc tgc tgt gtg ccc acg      2046
   Asn Ser Met Asp Pro Glu Ser Thr Pro Pro Thr Cys Cys Val Pro Thr
       455                 460                 465
   cgg ctg agt ccc atc agc atc ctc ttc att gac tct gcc aac aac gtg      2094
   Arg Leu Ser Pro Ile Ser Ile Leu Phe Ile Asp Ser Ala Asn Asn Val
   470                 475                 480                 485
   gtg tat aag cag tat gag gac atg gtc gtg gag tcg tgt ggc tgc agg      2142
   Val Tyr Lys Gln Tyr Glu Asp Met Val Val Glu Ser Cys Gly Cys Arg
                   490                 495                 500
   tagcagcact ggccctctgt cttcctgggt ggcacatccc aagagcccct tcctgcactc   2202
   ctggaatcac agaggggtca ggaagctgtg gcaggagcat ctacacagct tgggtgaaag   2262
   gggattccaa taagcttgct cgctctctga gtgtgacttg ggctaaaggc ccccttttat   2322
   ccacaagttc ccctggctga ggattgctgc ccgtctgctg atgtgaccag tggcaggcac   2382
   aggtccaggg agacagactc tgaatgggac tgagtcccag gaaacagtgc tttccgatga   2442
   gactcagccc accatttctc ctcacctggg ccttctcagc ctctggactc tcctaagcac   2502
   ctctcaggag agccacaggt gccactgcct cctcaaatca catttgtgcc tggtgacttc   2562
   ctgtccctgg gacagttgag aagctgactg ggcaagagtg ggagagaaga ggagagggct   2622
   tggatagagt tgaggagtgt gaggctgtta gactgttaga tttaaatgta tattgatgag   2682
   ataaaaagca aaactgtgcc t                                              2703
<210> 2
   <211> 501
   <212> PRT
   <213> homo sapiens
<400> 2
   Met Arg Leu Pro Lys Leu Leu Thr Phe Leu Leu Trp Tyr Leu Ala Trp
   1               5                   10                  15
   Leu Asp Leu Glu Phe Ile Cys Thr Val Leu Gly Ala Pro Asp Leu Gly
               20                  25                  30
   Gln Arg Pro Gln Gly Thr Arg Pro Gly Leu Ala Lys Ala Glu Ala Lys
           35                  40                  45
   Glu Arg Pro Pro Leu Ala Arg Asn Val Phe Arg Pro Gly Gly His Ser
       50                  55                  60
   Tyr Gly Gly Gly Ala Thr Asn Ala Asn Ala Arg Ala Lys Gly Gly Thr
   65                  70                  75                  80
   Gly Gln Thr Gly Gly Leu Thr Gln Pro Lys Lys Asp Glu Pro Lys Lys
                   85                  90                  95
   Leu Pro Pro Arg Pro Gly Gly Pro Glu Pro Lys Pro Gly His Pro Pro
               100                 105                 110
   Gln Thr Arg Gln Ala Thr Ala Arg Thr Val Thr Pro Lys Gly Gln Leu
           115                 120                 125
   Pro Gly Gly Lys Ala Pro Pro Lys Ala Gly Ser Val Pro Ser Ser Phe
       130                 135                 140
   Leu Leu Lys Lys Ala Arg Glu Pro Gly Pro Pro Arg Glu Pro Lys Glu
   145                 150                 155                 160
   Pro Phe Arg Pro Pro Pro Ile Thr Pro His Glu Tyr Met Leu Ser Leu
                   165                 170                 175
   Tyr Arg Thr Leu Ser Asp Ala Asp Arg Lys Gly Gly Asn Ser Ser Val
               180                 185                 190
   Lys Leu Glu Ala Gly Leu Ala Asn Thr Ile Thr Ser Phe Ile Asp Lys
           195                 200                 205
   Gly Gln Asp Asp Arg Gly Pro Val Val Arg Lys Gln Arg Tyr Val Phe
       210                 215                 220
   Asp Ile Ser Ala Leu Glu Lys Asp Gly Leu Leu Gly Ala Glu Leu Arg
   225                 230                 235                 240
   Ile Leu Arg Lys Lys Pro Ser Asp Thr Ala Lys Pro Ala Ala Pro Gly
                   245                 250                 255
   Gly Gly Arg Ala Ala Gln Leu Lys Leu Ser Ser Cys Pro Ser Gly Arg
               260                 265                 270
   Gln Pro Ala Ser Leu Leu Asp Val Arg Ser Val Pro Gly Leu Asp Gly
           275                 280                 285
   Ser Gly Trp Glu Val Phe Asp Ile Trp Lys Leu Phe Arg Asn Phe Lys
       290                 295                 300
   Asn Ser Ala Gln Leu Cys Leu Glu Leu Glu Ala Trp Glu Arg Gly Arg
   305                 310                 315                 320
   Ala Val Asp Leu Arg Gly Leu Gly Phe Asp Arg Ala Ala Arg Gln Val
                   325                 330                 335
   His Glu Lys Ala Leu Phe Leu Val Phe Gly Arg Thr Lys Lys Arg Asp
               340                 345                 350
   Leu Phe Phe Asn Glu Ile Lys Ala Arg Ser Gly Gln Asp Asp Lys Thr
           355                 360                 365
   Val Tyr Glu Tyr Leu Phe Ser Gln Arg Arg Lys Arg Arg Ala Pro Leu
       370                 375                 380
   Ala Thr Arg Gln Gly Lys Arg Pro Ser Lys Asn Leu Lys Ala Arg Cys
   385                 390                 395                 400
   Ser Arg Lys Ala Leu His Val Asn Phe Lys Asp Met Gly Trp Asp Asp
                   405                 410                 415
   Trp Ile Ile Ala Pro Leu Glu Tyr Glu Ala Phe His Cys Glu Gly Leu
               420                 425                 430
   Cys Glu Phe Pro Leu Arg Ser His Leu Glu Pro Thr Asn His Ala Val
           435                 440                 445
   Ile Gln Thr Leu Met Asn Ser Met Asp Pro Glu Ser Thr Pro Pro Thr
       450                 455                 460
   Cys Cys Val Pro Thr Arg Leu Ser Pro Ile Ser Ile Leu Phe Ile Asp
   465                 470                 475                 480
   Ser Ala Asn Asn Val Val Tyr Lys Gln Tyr Glu Asp Met Val Val Glu
                       485                 490                 495
   Ser Cys Gly Cys Arg
               500
<210> 3
   <211> 360
   <212> DNA
   <213> Homo sapiens
<220>
   <221> CDS
   <222> (1)..(360)
   <223> human mature monomeric GDF-5
<400> 3
   gca cca cta gca act cgt cag ggc aag cga ccc agc aag aac ctt aag       48
   Ala Pro Leu Ala Thr Arg Gln Gly Lys Arg Pro Ser Lys Asn Leu Lys
   1               5                   10                  15
   gct cgc tgc agt cgg aag gca ctg cat gtc aac ttc aag gac atg ggc       96
   Ala Arg Cys Ser Arg Lys Ala Leu His Val Asn Phe Lys Asp Met Gly
               20                  25                  30
   tgg gac gac tgg atc atc gca ccc ctt gag tac gag gct ttc cac tgc      144
   Trp Asp Asp Trp Ile Ile Ala Pro Leu Glu Tyr Glu Ala Phe His Cys
           35                  40                  45
   gag ggg ctg tgc gag ttc cca ttg cgc tcc cac ctg gag ccc acg aat      192
   Glu Gly Leu Cys Glu Phe Pro Leu Arg Ser His Leu Glu Pro Thr Asn
       50                  55                  60
   cat gca gtc atc cag acc ctg atg aac tcc atg gac ccc gag tcc aca      240
   His Ala Val Ile Gln Thr Leu Met Asn Ser Met Asp Pro Glu Ser Thr
   65                  70                  75                  80
   cca ccc acc gcc tgt gtg ccc acg cga ctg agt ccc atc agc atc ctc      288
   Pro Pro Thr Ala Cys Val Pro Thr Arg Leu Ser Pro Ile Ser Ile Leu
                   85                  90                  95
   ttc att gac tct gcc aac aac gtg gtg tat aag cag tat gag gac atg      336
   Phe Ile Asp Ser Ala Asn Asn Val Val Tyr Lys Gln Tyr Glu Asp Met
               100                 105                 110
   gtc gtg gag tcg tgt ggc tgt agg                                      360
   Val Val Glu Ser Cys Gly Cys Arg
           115                 120
<210> 4
   <211> 120
   <212> PRT
   <213> Homo sapiens
<400> 4
   Ala Pro Leu Ala Thr Arg Gln Gly Lys Arg Pro Ser Lys Asn Leu Lys
   1               5                   10                  15
   Ala Arg Cys Ser Arg Lys Ala Leu His Val Asn Phe Lys Asp Met Gly
               20                  25                  30
   Trp Asp Asp Trp Ile Ile Ala Pro Leu Glu Tyr Glu Ala Phe His Cys
           35                  40                  45
   Glu Gly Leu Cys Glu Phe Pro Leu Arg Ser His Leu Glu Pro Thr Asn
       50                  55                  60
   His Ala Val Ile Gln Thr Leu Met Asn Ser Met Asp Pro Glu Ser Thr
   65                  70                  75                  80
   Pro Pro Thr Ala Cys Val Pro Thr Arg Leu Ser Pro Ile Ser Ile Leu
                   85                  90                  95
   Phe Ile Asp Ser Ala Asn Asn Val Val Tyr Lys Gln Tyr Glu Asp Met
               100                 105                 110
   Val Val Glu Ser Cys Gly Cys Arg
           115                 120

## Description

The present invention is directed to novel drug delivery devices and pharmaceutical compositions containing growth and differentiation factor proteins. Said devices and compositions are specifically designed to accelerate tissue regeneration and wound healing processes of mammalian tissues. The invention is especially useful for the supportive therapy of diabetic wounds and ulcers.

GDF-5 (Hötten et al. 1994, Biochem. Biophys Res. Commun. 204, 646-652) is a morphogen which has been shown to promote cell proliferation, differentiation and/or tissue formation in several tissues. The protein is also known as morphogenic protein MP52, bone morphogenetic protein-14 (BMP-14) or cartilage-derived morphogenetic protein-1 (CDMP-1). GDF-5 is closely related to GDF-6 and GDF-7. These three proteins form a distinct subgroup of the TGF-ß superfamily, thus displaying comparable biological properties and an extraordinary high degree of amino acid sequence identity (see i.e. Wolfman et al. 1997, J. Clin. Invest. 100, 321-330). All family members are initially synthesized as larger precursor proteins which subsequently undergo proteolytic cleavage at a cluster of basic residues approximately 110-140 amino acids from the C-terminus, thus releasing the C-terminal mature protein parts from the N-terminal prodomain. The mature polypeptides are structurally related and contain a conserved bioactive domain comprising six or seven canonical cysteine residues which is responsible for the characteristical three-dimensional "cystine-knot" motif of these proteins. Native GDF-5 related proteins are homodimeric molecules and act mainly through interaction with specific receptor complexes which are composed of type I and type II serine/threonine receptor kinases. The receptor kinases subsequently activate Smad proteins, which then propagate the signals into the nucleus to regulate target gene expression.

It has repeatedly been demonstrated that members of the GDF-5/-6/-7 subgroup are primarily important inducers and regulators of bone and cartilage (Cheng et al. 2003, J. Bone & Joint Surg. 85A, 1544-1552; Settle et al. 2003, Developm. Biol. 254, 116-130). GDF-5 is a natural growth factor in the nervous system (see for example WO 97/03188; Krieglstein et al., (1995) J. Neurosci Res. 42, 724-732; Sullivan et al., (1997) Neurosci Lett 233, 73-76; Sullivan et al. (1998), Eur. J. Neurosci 10, 3681-3688). Furthermore, it is e.g. useful for the modulation of skin related tissue growth (WO 02/076494; Battaglia et al. 2002, Trans. Orthop. Res. Soc. 27, 584), and for induction of angiogenic processes (Yamashita et al. 1997, Exp. Cell Res. 235, 218-26).

After discovery of their unique tissue inductive activities, growth factor proteins such as GDF-5 have been successfully applied in therapeutic research and regenerative surgery, in which they promote and assist the natural healing process of various damaged tissues, either alone or in combination with specific matrix materials. Although several pharmaceutical compositions comprising biologically active mature GDF-5 related proteins have been developed (see e.g. WO96/33215), formulation and handling of GDF-5 are nevertheless still problematic because the mature protein tends to interact with a couple of solid materials and shows exceptional poor solubility under physiological conditions. A pH-dependent solubility profile of mature GDF-5/MP52 (shown i.e. in EP 1 462 126) reveals that the protein starts precipitating in aqueous solutions with a pH above 4.25 and becomes almost unsoluble between pH 5 and pH 9.

According to these facts, previous attempts to formulate stable liquid or gel-like GDF-5 compositions have faced serious problems. Limited success was achieved for special applications only. EP 1 462 126 which describes materials for bone regeneration succeeded in improving the protein solubility profile slightly by using solvents with low ionic strength. WO2008/049588 describes a lipid nanosphere formulation which is capable of delivering significant quantities of GDF-5 related proteins in vivo along neuronal pathways. However, the combination of GDF-5 related proteins with particular materials remains challenging. Nevertheless there is still a great need to develop novel methods and pharmaceutical compositions for the efficient administration of such proteins under physiological conditions.

US 6,669,980 B2 discloses a method for coating a medical device, wherein the medical devices are coated with thermoplatic polymers and comprise also some GDF-5 related proteins such as Bone Morphogenic Proteins.

FR 2919188 A1 discloses a complex of an amphiphilic polymer and a BMP. Nishant Kumar Jain et al., "Effect of trehalose on protein structure", Protein Science, Vol. 18, No. 1, 1 January 2009, DOI: 10.1002/pro.3 pertains to the effect of trehalose on protein structure.

For wound healing purposes, both lotion-like and solid surgical dressings of various forms, sizes and materials have been developed which are primarily designed to ensure wound closure under semi-sterile conditions. Several of these dressings are made up of organic materials such as e.g. collagens whereas other devices are composed of synthetic components such as e.g. amorphous thermoplastic polymers. Some wound dressings of the most advanced generation feature additional drug delivery functions; they are capable of administering bioactive substances such as antibiotics or cytokines like epidermal growth factor (EGF) or platelet-derived growth factor (PDGF/Becaplermin). For example, genetically engineered PDGF is commercially available under the brand name Regranex® as a topical (0.01%) wound healing gel which has been approved for the treatment of diabetic foot ulcers that extend into the subcutaneous tissue or beyond. Especially desirable for wound healing and other tissue regeneration purposes are new formulations, surgical dressings and drug delivery devices through which bioactive substances are being delivered to the human body in a controlled manner, thus precisely satisfying the high demand of the body. It is therefore an object of the invention to improve the therapeutic usability of GDF-5 and related proteins by providing stable and non-toxic growth factor compositions which are applicable in combination with synthetic wound healing materials and devices described hereinafter.

### Definitions:

In order to avoid misunderstandings and ambiguities, some frequently used terms herein are defined and exemplified as follows:

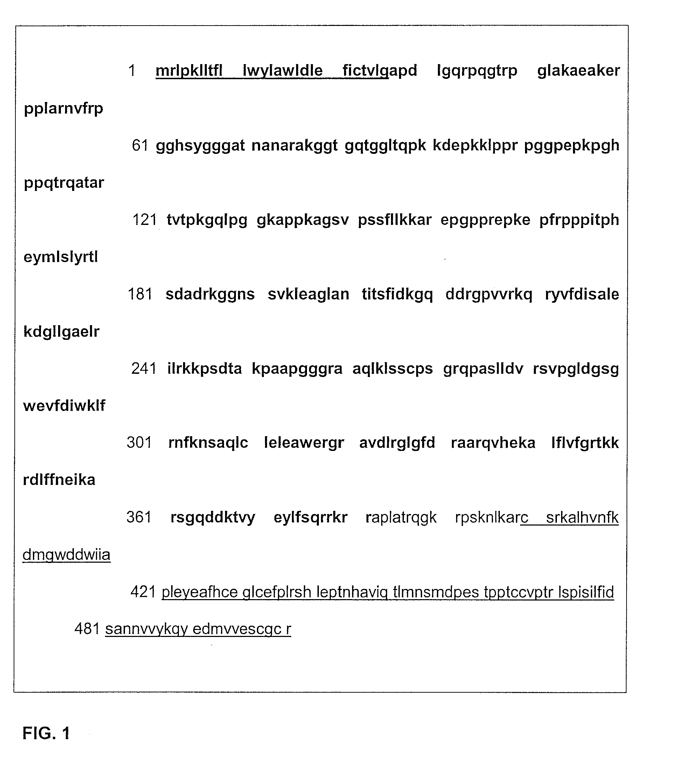

The term "cystine-knot domain" as used herein means the well known and conserved cysteine-rich amino acid region which is present in the mature parts of TGF-beta superfamily proteins such as i.e. human GDF-5 and forms a three-dimensional protein structure known as cystine-knot. In this domain the respective location of the cysteine residues to each other is important and is only allowed to vary slightly in order not to lose the biological activity. It has been demonstrated that the cystine-knot domain alone is sufficient for the biological function of the protein (Schreuder et al. (2005), Biochem Biophys Res Commun. 329, 1076-86). Consensus sequences for cystine-knot domains are well known in the state of the art. According to the definition defined herein the cystine-knot-domain of a protein starts with the first cysteine residue participating in the cystine-knot of the respective protein and ends with the residue which follows the last cysteine participating in the cystine-knot of the respective protein. For example, the cystine-knot domain of the human GDF-5 precursor protein (SEQ ID NO: 2) consists of the amino acids 400-501 (see also FIG. 1).

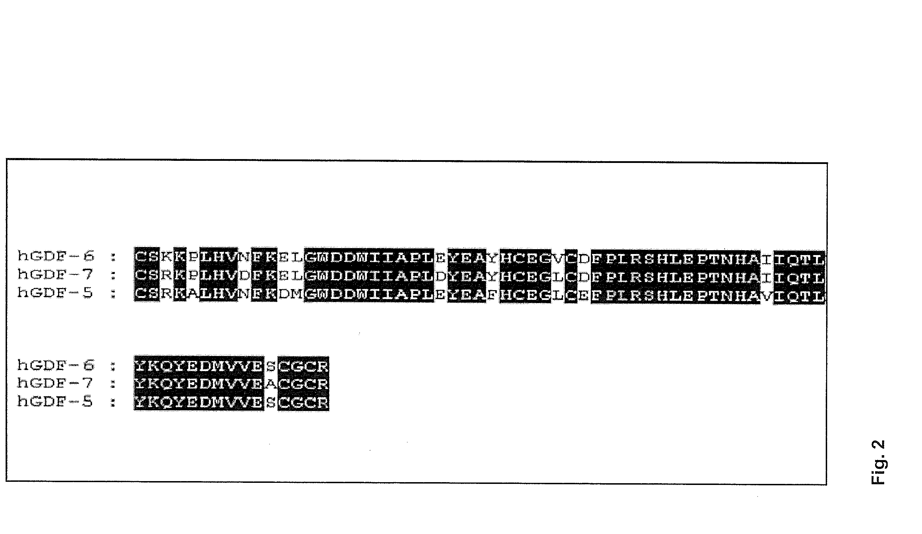

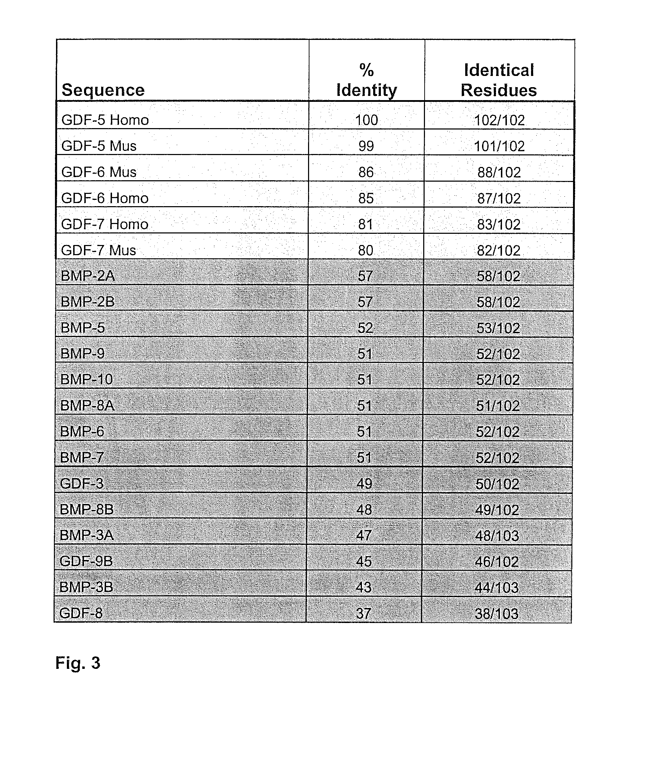

The term "GDF-5-related protein" as used herein means any naturally occurring or artificially created protein which comprises a cystine-knot-domain with an amino acid identity of at least 60% to the 102 aa cystine-knot domain of human GDF-5 (amino acids 400-501 of SEQ ID NO: 2). This term includes proteins belonging to the group of GDF-5, GDF-6 and GDF-7 proteins from vertebrate or mammalian species as well as recombinant variants thereof as long as these proteins show the above mentioned percentage of identity with the cystine-knot domain of human GDF-5. The limiting value of 60% is well suitable to separate members of the GDF-5/-6/-7 group of proteins as well as variants thereof from further proteins such as other GDFs and BMPs. A comparison of the 102 aa cystine-knot-domains of human GDF-5, human GDF-6 and human GDF-7 (see FIG. 2) reveals the high grade of amino acid identity between these proteins. Human GDF-6 shares 87 (85%) and human GDF-7 shares 83 (81%) identical residues with the cystine-knot-domain of human GDF-5. The respective domains of GDF-5/-6/-7 molecules from other vertebrate and mammalian species which have been identified so far also show very high identity percentages of at least 75% (between 79% and 99%), when compared with human GDF-5. In contrast, GDFs and BMPs not belonging to the GDF-5/-6/-7 subgroup display much lower identity values below 60% (see FIG. 3)

The determination of corresponding amino acid positions in related amino acid sequences as well as the calculation of percentages of identity between can be easily performed with the help of well known alignment algorithms and optionally computer programs using these algorithms. For example, the amino acid identities in this patent application (i.e. FIG. 2) have been calculated by aligning sequences with the freeware program ClustalX (Version 1.81) with default parameters and subsequent counting of identical residues by hand. Default settings for pairwise alignment (slow-accurate) are: gap opening parameter: 10.00; gap extension parameter 0.10; Protein weight matrix: Gonnet 250. The ClustalX program is described in detail in Thompson,J.D., Gibson,T.J., Plewniak,F., Jeanmougin,F. and Higgins,D.G. (1997): The ClustalX windows interface: flexible strategies for multiple sequence alignment aided by quality analysis tools. Nucleic Acids Research 24:4876-4882. ClustalX is a windows interface for the ClustalW multiple sequence alignment program and is i.e. available from various sources, i.e. by anonymous ftp from ftp-igbmc.u-strasbg.fr, ftp.embl-heidelberg.de, ftp.ebi.ac.uk or via download from the following webpage: http://www-igbmc.u-strasbg.fr/BioInfo/. The ClustalW program and algorithm is also described in detail in Thompson, J.D., Higgins, D.G. and Gibson, T.J. (1994): CLUSTALW: improving the sensitivity of progressive multiple sequence alignment through sequence weighting, positions-specific gap penalties and weight matrix choice. Nucleic Acids Research 22:4673-4680.

The term "variant" as used herein means any of the following polypeptides:
a) biologically active fragments of a protein, preferably at least comprising the cystine-knot domain;
b) biologically active protein constructs which contain additional sequences ( either with or without adding biological functions) in excess to the original sequence of the protein or constructs which contain amino acid substitutions;
c) any combination of a) and b).

The term "medical device" refers to any material (single or composite), instrument or apparatus or, whether used alone or in combination, is intended to be used by the manufacturer for the purpose of treatment or alleviation of disease or injury. This includes devices that do not achieve its principal intended action in or on the human body by pharmacological, immunological or metabolic means, but which may be assisted in its function by such means.

The term "amorphous thermoplastic polymers" refers to synthetic polymeric materials having a randomly ordered molecular structure and which can be formed into a load-bearing shape. For better understanding: high temperature materials are divided into two main categories - semi-crystalline and amorphous - based on their difference in molecular structure. Solid organic compounds consisting of ordinary small molecules tend to be crystalline, that is, the molecules pack themselves in regular three-dimensional arrays. Polymers are different; they can be amorphous (totally lacking positional order on the molecular scale, thereby creating local regions with different charge) or semi-crystalline (containing varying percentages of crystalline regions in the same sample). Amorphous thermoplastic polymers have characteristic attributes: they do not have a sharp melt point; they change viscosity when heated and they are isotropic in flow. As a result, amorphous materials typically exhibit lower mold shrinkage and fewer tendencies to warp than the semi-crystalline materials. The term "amorphous thermoplastic polymers" comprises polymeric materials such as polysulfones (PSU), polyethersulfones (PES), polyphenylsulfones (PPSU), polystyrenes, polyetherimides, polyarylates, polyvinylchlorides, polyamides (amorphous), polymethylmethacrylates, polyamideimides, and acrylonitrile butadiene styrenes.

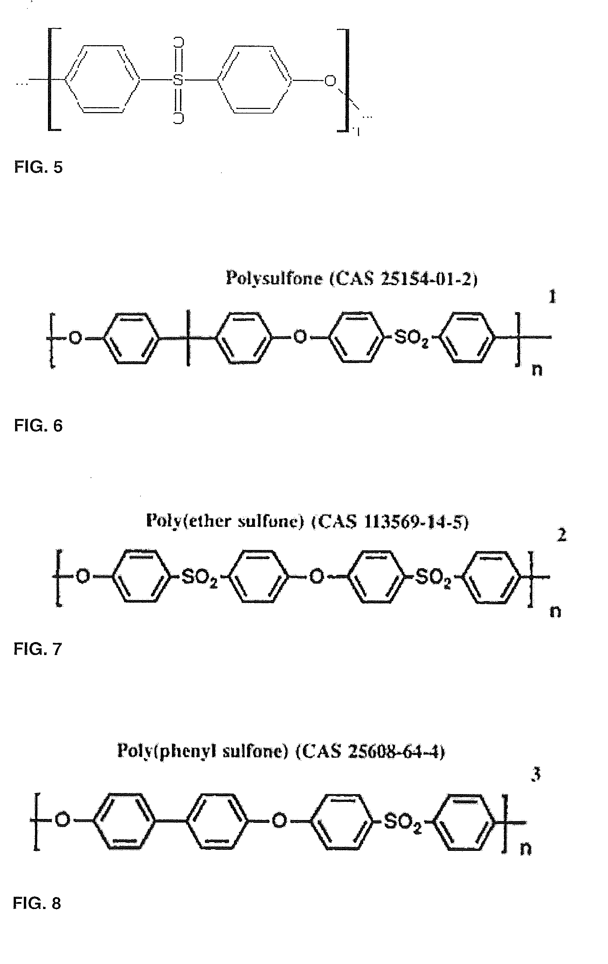

The term "polysulfone-based plastics" denotes a group of thermoplastic polymers containing the subunit aryl-SO₂-aryl, the defining feature of which is the sulfone group. The repeating unit of these polymers is shown in FIG. 5. The material allows easy manufacturing of membranes, with reproducible properties and controllable size of pores down to 40 nanometres. Polysulfone-based plastics can be reinforced with glass fibers and are also used as a copolymer. The group of "polysulfone-based plastics" contains the chemically related substances polysulfone (also spelled polysulphone, abbreviated PSU), polyethersulfone (also spelled polyethersulphone, abbreviated PES), and polyphenylsulfone (also spelled polyphenylsulphone, abbreviated PPSU).

The term "polysulfones" (PSU) denotes thermoplastic polymers (CAS identifier 25154-01-2) with the repeating unit shown in FIG. 6.

The term "polyethersulfones" (PES) denotes thermoplastic polymers (CAS identifier 113569-14-5) with the chemical formula Poly(oxy-1,4-phenylsulfonyl-1,4-phenyl). The substance is closely related to polysulfones and polyphenylsulfones. A repeating unit of PES is shown in FIG. 7

The term "polyphenylsulfones" (PPSU) denotes thermoplastic polymers (CAS identifier 25608-64-4) with the repeating unit shown in FIG. 8. The substance is closely related to polysulfones and polyethersulfones.

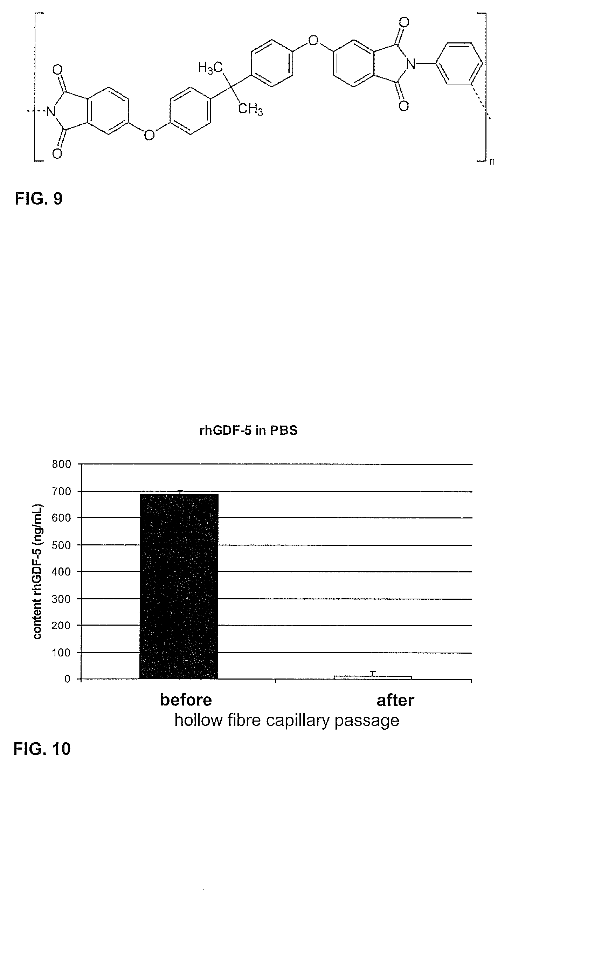

The term "polyetherimides" (PEI) denotes thermoplastic polymers (CAS identifier 61128-46-9) with the repeating unit shown in FIG. 9. The molecular formula of repeating unit of PEI is C₃₇H₂₄O₆N₂ and the molecular weight is 592 g/mol.

The term "carboxymethyldextran" (CMD) denotes polyanionic derivatives of dextran with varying amounts of carboxymethyl groups attached by a stable O-ether linkage. CMDs of various molecular weight (common molecular weights of CMD are e.g. 10 000, 40 000, 1 000 000) are commercially available. According to this definition and the invention disclosed hereinafter, the CMD might be also further chemically modified (e.g. by carrying additional binding groups), as long as the molecule is still capable of preventing the molecular stickiness between GDF-5 related proteins and the solid materials described in this patent application, e.g. amorphous thermoplastic polymers, polyvinylpyrrolidon or biodegradable materials such as e.g. woven or nonwoven collagen, gelatine, polylactide (PLA), polyglycolide (PGA), polycaprolactone (PCL) polylactid, dextran, hyaluronic acid and chitosan or combinations thereof.

The term "biological activity" denotes the activity of compounds, including, e.g., a GDF-5-related protein as measured by the common in vitro alkaline phosphatase assay (ALP), e.g. as described in example 8 or in Takuwa et al. (1989), Am. J. Physiol. 257, E797-E803). Suitable cell lines which may be used in such ALP assay are e.g. ATDC-5 or MCHT 1/26 cells.

In brief, the present invention describes a significant improvement of the medical device-mediated delivery of bioactive GDF-5 related proteins towards sites where tissue regeneration is desirable. More precisely, the invention enables the use and delivery of these growth factor proteins in combination with medical devices made of certain synthetic and natural materials.

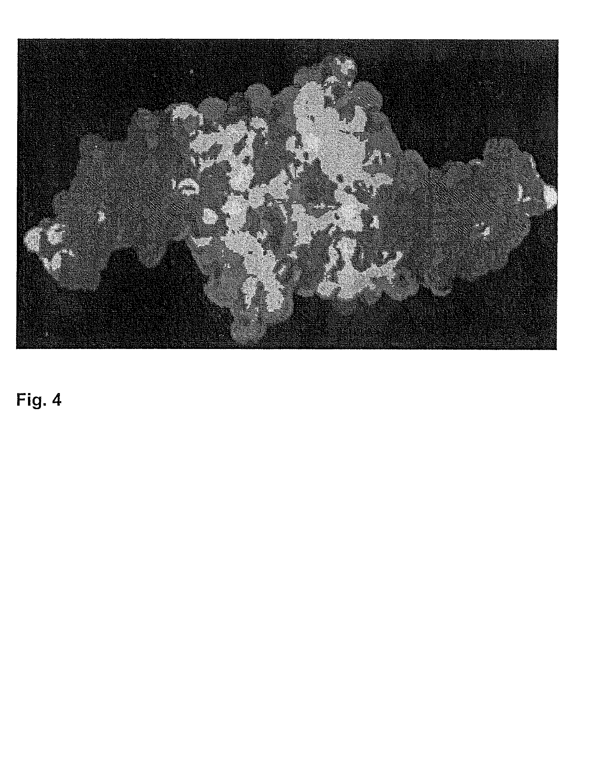

GDF-5 and the closely related proteins GDF-6 and GDF-7 feature an uncommon surface charge distribution pattern. In contrast to most other hydrophobic proteins, the surface of GDF-5 is not predominantly composed of hydrophobic amino acids such as alanine, valine and leucine and features an uncommon charge distribution effect which is shown in FIG. 4. Over a wide pH range, the surface of GDF-5 comprises large regions of varying length with opposite charge. These protein parts attract each other and thereby seem to initiate coagulation and precipitation of the growth factor molecule. The uncommon charge distribution of GDF-5 related proteins also increases the adhesion to several solid materials with alternating charge patterns or other uncommon substance characteristics.

The adhesion between GDF-5 related proteins and solid materials is e.g. noticeable in case of polyvinylpyrrolidon and certain biodegradable materials such as e.g. woven or nonwoven collagen, gelatine, polylactide (PLA), polyglycolide (PGA), polycaprolactone (PCL) polylactid, dextran, hyaluronic acid and chitosan or combinations thereof. However, said unwanted adhesion effect is particularly strong if certain amorphous thermoplastic polymers are used in combination with GDF-5, for example if they are part of a medical drug delivery device. Due to their amorphous character with the polymeric string not regularly folded but disordered, these synthetic polymers - although consisting of high numbers of identical repeating units (see e.g. Fig. 5 to 9) - exhibit a randomly ordered molecular structure with both hydrophobic and more polar/hydrophilic regions. There is evidence that inter alia this attribute, in combination with further topographical features, causes the strong molecular stickiness in case of a direct contact with a solution comprising GDF-5 related proteins.

According to the present invention, said molecular stickiness between GDF-5 related proteins and various materials, particularly amorphous thermoplastic polymers which are preferably selected from the group consisting of polysulfone-based plastics and polyetherimides and which are most preferably selected from the group consisting of polysulfones (PSU), polyethersulfones (PES) and polyphenylsulfones (PPSU) can be efficiently prevented with the help of certain organic additives belonging to the group of carbohydrates.

In a first embodiment of the invention, said organic additive is trehalose. Trehalose, also known as mycose or tremalose, is a natural alpha-linked disaccharide formed by an α,α-1,1-glucoside bond between two α-glucose units. More precisely, addition of a defined amount of trehalose to a solution of one or more GDF-5 related proteins causes an efficient blocking of adhesional interactions between GDF-5 related proteins and amorphous thermoplastic polymers. According to the experiments performed by the inventor, it is required for a sufficient blocking effect that the solution of one or more GDF-5 related proteins contains between 0.001 and 5% trehalose. Particularly good effects could be observed with a solution containing between 0.01 and 3% trehalose and even better effects with a solution containing between 0.1 and 2% trehalose. Best effects were seen if said solution contained between 0.5 and 1% trehalose.

In a second embodiment of the invention, said organic additive is dextran sulphate or carboxymethyldextran sulfate, which can be used in most cases as a replacement for trehalose. Dextran sulfate is a long-chain (with variable length) polymer of glucose containing 17-20% sulphur. It is preferred that a dextran sulfate with an average molecular weight between 100 and 10 000, most preferably 500 (e.g. Amersham 17-0340-01), is used. Good blocking effects were seen if the protein solution contained between 0.001 and 5% dextran sulfate or carboxymethyldextran sulfate, preferably between 0.01 and 3% dextran sulfate or carboxymethyldextran sulfate , more preferably between 0. 1 and 2% dextran sulfate or carboxymethyldextran sulfate and most preferably between 0.5 and 1 % dextran sulfate or carboxymethyldextran sulfate.

In a third embodiment of the invention, said organic additive is carboxymethyl dextran which is chemically closely related to carboxymethyldextran sulphate. Good blocking effects were seen if the protein solution contained between 0.001 and 5% carboxymethyl dextran, preferably between 0.01 and 3% carboxymethyl dextran, more preferably between 0. 1 and 2% carboxymethyl dextran and most preferably between 0.5 and 1 % carboxymethyl dextran.

As e.g. shown in example 4, even very low concentrations (0.001 %) of either trehalose or dextran sulfate prevented the binding of at least 75% of rhGDF-5 to hollow fibre capillaries made of PES or other amorphous thermoplastic polymers. With higher carbohydrate concentrations, nearly 100 % of the injected rhGDF-5 could be detected after hollow fibre capillary passage. Thus, in certain embodiments of the invention, the concentration of trehalose, dextran sulfate, carboxymethyl dextran sulfate or carboxymethyl dextran prevents the binding of at least 75%, at least 85%, at least 90%, at least 95% or at least 99% of dissolved GDF-5 related proteins to amorphous thermoplastic polymers.

According to the invention, the apparatus or medical device which has a storage and/or delivery function of GDF-5 related proteins and which comprises one or more amorphous thermoplastic polymers may have different forms, shapes, styles or designs. For example, the medical drug delivery device shown in example 9 is a hollow fibre capillary device made of polyethersulfone (PES). Generally preferred are medical devices containing one or more structural elements for the storage or delivery of GDF-5 related proteins which are selected from the group consisting of a hollow fibre, a capillary, a tubing, a tank, a container, a mesh, a spongy element, and/or a membrane.

The parts and embodiments of the invention described in this patent specification apply to all naturally occurring or artificially designed proteins which are very closely related to human growth/differentiation factor 5 (hGDF-5). The term "GDF-5-related proteins" includes functionally similar proteins belonging to the group of vertebrate GDF-5, GDF-6 and GDF-7 proteins as well as recombinant variants thereof. Due to an extraordinary high degree of amino acid sequence identity (see FIG. 2), this group of proteins exhibits comparable biological properties. Common feature of all GDF-5-related proteins is the occurrence of a bioactive cystine-knot-domain with an amino acid identity of at least 60% to the 102 aa cystine-knot domain of human GDF-5/SEQ ID NO: 2 which is sufficient for the biological function of the protein. As can be seen from FIG. 3, the preferred limiting value of 60% separates members of the GDF-5/-6/-7 group from more distantly related GDFs and BMPs. Especially preferred proteins display amino acid identities of at least 70%, 80% or 90% to the 102 aa cystine-knot domain of human GDF-5.

Non-limiting examples for vertebrate and mammalian GDF-5-related proteins are precursors and mature proteins of human GDF-5 (disclosed as MP52 in WO95/04819 and as human GDF-5 in Hötten et al. 1994, Biochem. Biophys Res. Commun. 204, 646-652), recombinant human (rh) GDF-5/MP52 (WO96/33215), MP52 Arg (WO97/06254); HMW human MP52s (WO97/04095), CDMP-1 (WO96/14335), mouse (Mus musculus) GDF-5 (US 5,801,014), rabbit (Oryctolagus cuniculus) GDF-5 (Sanyal et al. 2000, Mol Biotechnol. 16, 203-210), chicken (Gallus gallus) GDF-5 (NCBI accession no. NP_989669), african clawed frog (Xenopus laevis) GDF-5 (NCBI accession no. AAT99303), monomeric GDF-5 (WO 01/11041 and WO 99/61611), human GDF-6/BMP-13 (US 5,658,882), mouse GDF-6 (NCBI accession no NP_038554), GDF-6/CDMP-2 (WO96/14335), human GDF-7/BMP-12 (US 5,658,882), mouse GDF-7 (NCBI accession no AAP97721), GDF-7/CDMP-3 (WO96/143335). Covered by the invention are also GDF-5-related proteins having additional mutations such as substitutions, additions and deletions, as long as these additional mutations do not completely abolish the biological protein activity. Some preferred variants are mutants of GDF-5-related proteins with improved biological activity. For example, one or more residues which are normally present in the human GDF-5 precursor protein (see FIG. 1) are substituted in these mutants by other amino acids: the arginine at position 438 of the human GDF-5 precursor is replaced by glycine, alanine, valine, leucine, isoleucine, methionine or asparagines; and/or serine 439 is replaced by aspartic acid, glutamic acid, glycine, leucine, or isoleucine; and/or asparagine 445 is replaced by serine or threonine. In another high activity mutant, methionine 453 and/or methionine 456 are replaced by alanine, valine, or isoleucine. Also of special interest are mutants in which leucine 441 is replaced by proline.

In general, the present invention can be applied in all situations in which storage and/or delivery of the above mentioned recombinant and wild-type GDF-5 forms in combination with medical devices made of synthetic polymeric compounds are useful. Thus, the present invention can be used to facilitate the regeneration of various tissues and organs. For example, GDF-5 is considered to be a very effective promoter of bone and cartilage formation as well as connective tissue formation (see for example WO 95/04819, Hötten et al. 1996, Growth Factors 13, 65-74; Storm et al. 1994, Nature 368, 639-643; Chang et al. 1994, J. Biol. Chem. 269, 28227-28234) and formation of connective tissue attachment (EP 0 831 884). In this context, GDF-5 is useful for applications concerning the joints between skeletal elements (see for example Storm & Kingsley 1996, Development 122, 3969-3979). One example for connective tissue is tendon and ligament (Wolfman et al. 1997, J. Clin. Invest. 100, 321-330; Aspenberg & Forslund 1999, Acta Orthop Scand 70, 51-54; WO 95/16035). The protein is helpful for meniscus and spinal/intervertebral disk repair (Walsh et al. 2004, Spine 29, 156-63) and spinal fusion applications (Spiro et al. 2000, Biochem Soc Trans. 28, 362-368). GDF-5 can be beneficially applied in tooth (dental and periodontal) applications (see for example WO 95/04819; WO 93/16099; Morotome et al. 1998, Biochem Biophys Res Comm 244, 85-90) such as the regeneration of dentin or periodontal ligament. GDF-5 is also useful in wound repair of any kind. It is also beneficial for promoting tissue growth in the neuronal system and survival of e.g. dopaminergic neurons. In this context, GDF-5 can be used for treating neurodegenerative disorders like e.g. Parkinson's disease and possibly also Alzheimer's disease or Huntington chorea tissues (see for example WO 97/03188; Krieglstein et al., (1995) J. Neurosci Res. 42, 724-732; Sullivan et al., (1997) Neurosci Lett 233, 73-76; Sullivan et al. (1998), Eur. J. Neurosci 10, 3681-3688). GDF-5 allows to maintain nervous function or to retain nervous function in already damaged tissues. GDF-5 is therefore considered to be a generally applicable neurotrophic factor. It is also useful for diseases of the eye, in particular retina, cornea and optic nerve (see for example WO 97/03188; You et al. (1999), Invest Opthalmol V is Sci 40, 296-311), for hair growth and the treatment and diagnosis of skin related disorders (WO 02/076494; Battaglia et al. 2002, Trans. Orthop. Res. Soc. 27, 584), and for induction of angiogenesis (Yamashita et al. 1997, Exp. Cell Res. 235, 218-26).

As such, a preferred indication in which the present invention can be applied is wound healing. The invention is especially suited to facilitate the treatment of burns, skin lesions, skin injuries or skin grafts, diabetic wounds and diabetic ulcers, e. g. diabetic foot ulcer.

Further non-limiting examples in which the present invention can be applied are the prevention or therapy of diseases associated with bone and/or cartilage damage or affecting bone and/or cartilage disease, or generally situations, in which cartilage and/or bone formation is desirable or for spinal fusion, prevention or therapy of damaged or diseased tissue associated with connective tissue including tendon and/or ligament, periodontal or dental tissue including dental implants, neural tissue including CNS tissue and neuropathological situations, tissue of the sensory system, liver, pancreas, cardiac, blood vessel, renal, uterine and thyroid tissue, mucous membranes, endothelium, epithelium, for promotion or induction of nerve growth, tissue regeneration, angiogenesis, induction of proliferation of progenitor cells and/or bone marrow cells, for maintenance of a state of proliferation or differentiation for treatment or preservation of tissue or cells for organ or tissue transplantation, for integrity of gastrointestinal lining, for treatment of disturbances in fertility, contraception or pregnancy. Diseases concerning sensory organs like the eye are also to be included in the preferred indication of the pharmaceutical composition according to the invention. As neuronal diseases again Parkinson's and Alzheimer's diseases can be mentioned as examples.

The biological activities of GDF-5-related proteins can be easily determined with the help of established test systems. Most useful and preferred is a common in vitro test known as alkaline phosphatase (ALP) assay (Takuwa et al. 1989, Am. J. Physiol. 257, E797-E803), which is also described in example 8. GDF-5-related proteins have been demonstrated to increase alkaline phosphatase activity i.e. in ROB-C26 cells (Yamaguchi et al. 1991, Calcif. Tissue Int. 49, 221-225) as described in WO95/04819, in embryonic ATDC5 cells (Riken Gene Bank, ROB 0565), in mouse stromal MCHT-1/26 cells, and in HPDL cells as shown in Nakamura et al. 2003, J. Periodontal Res. 38,597-605.

The concentrations of GDF-5-related proteins in the compositions of the invention should be chosen in dependency on the mode and period of application. Basically, GDF-5-related proteins are highly potent cytokines which are capable of eliciting effects even in exiguous quantities. As easily determinable with the help of different biological assay systems such as i.e. the alkaline phosphatase assay described herein, a concentration of 0.1 pg GDF-5 per ml of the respective solution is sufficient to cause biological actions. Accordingly, low concentrations, i.e. ranging from 0.1 pg/ml to 1 ng/ml or less, are preferred if the compositions of the invention are repeatedly administered. However, maximum effects are achievable with higher growth factor concentrations of 1 - 100 ng/ml. An independent dose response analysis of GDF-5 action utilizing a wide range of serial dilutions (0.3 - 80 ng/ml, Farkas et al. 1997, Neurosci. Lett. 236, 120-122) gave optimal results at a concentration of 20 ng GDF-5 per ml. *In vivo* skin models commonly use high doses of 1 - 10 µg/ml. Therefore, in a preferred embodiment of the invention, the compositions of the invention contain GDF-5 related proteins in concentrations of between 0.1 pg/ml and 10 µg/ml. Preferred total doses of GDF-5 related proteins in case of one time administrations range from 10 ng to 10 µg.

A further aspect of the invention relates to additional ingredients and components of the formulations disclosed herein.

Most administration procedures require compositions having a nearly physiological pH. Unfortunately, GDF-5 and the closely related proteins GDF-6 and GDF-7 feature poor solubility at pH values between pH 4 and pH 9. Although GDF-5-related proteins may also be administered by means of aqueous solutions having a pH around or below 4, another way to overcome the solubility problem is the adhesion to a specific colloidal drug carrier of very small particle size. The interaction between growth factor and the selected microstructured carrier efficiently prevents the undesired coagulation of the protein at slightly acid/basic and even at neutral pH. In principle, various colloidal carriers known in the art may be utilized for cosmetic and pharmaceutical compositions. Suitable carriers are extensively described in the literature (see i.e. Barrat et al. 2003: Colloidal drug carriers: achievements and perspectives. Cell. mol. life sci. 60, 21-37). Among several others, frequently used colloidal carriers are i.e. liposomes, mixed micelles, microemulsions, lipid microparticles and polymeric nanoparticles. Lipid microparticles may be further divided into lipid nanospheres (diameter below 200 nm) and micospheres (diameter 0.2 - 100 µm). Creation and testing of these and other colloidal drug carriers is a routine matter which can be done without undue burden.

In addition, the formulation might comprise natural and synthethic lipids. All kinds of natural and synthetic oils/lipids can be used as long as they are biocompatible, for example synthetic oils or saturated esters such as ethyl palmitate, isopropyl palmitate, alkyl myristates such as those of isopropyl, butyl and cetyl, hexyl stearate, triglycerides (i.e. of octanoic or decanoic acids, medium chained tryglycerides such as Miglyol® 812), cetyl ricinoleate, stearyl octanoate (purcelllin oil) and hydrogenated polyisobutene, or natural oils such as e.g. cottonseed, soybean, sesame, sunflower, safflower, olive, avocado, peanut, walnut, almond and hazelnut oil.

The formulation might also comprise emulsifying agents, for example phospholipids such as phosphatidylserine, phosphatidylcholine or phosphatidylethanolamine, distilled monoglycerides, mono- & diglycerides, acetic acid esters of monoglycerides, organic esters of monoglycerides, sorbitan esters of fatty acids, propylene glycol esters of fatty acids and polyglycerol esters of fatty acids.

Other bioactive protein(s) in addition to GDF-5-related proteins might also be part of the compositions of the invention. It has been shown that TGF-ß increases the size of regenerated dermis and stabilizes the dermoepithelial junction (Fitzpatrick and Rosen, J. Cosmet. Laser Ther, 5: 25-34 (2003)). A cocktail (TNS Recovery Complex, SkinMedica, Inc. Carlsbad, CA, USA) containing seven cytokines (VEGF, IL-6 and -8, HGF, PDGF-a, GCSF, and TGF-ß1) derived from neonatal foreskin fibroblasts was tested in a multicenter study. Evaluation showed improvement in skin texture, and decreased wrinkling (Rokhsar, C.K. et al., Dermatol. Surg. 31: 1166-1178 (2005)). Recombinant epidermal growth factor (ReVive Skincare); and N-furfuryladenine (kinetin) plant growth factor are also on the market. All these proteins may be used together with the GDF-5-related proteins of the invention. Other proteins which act synergistically if combined with GDF-5-related proteins are disclosed in the literature/patents, i.e. in WO 99/15191. Preferred are neurotrophins, hedgehog proteins and proteins of the transforming growth factor family, including but not limited to TGF-alpha's, TGF-beta's, activins, BMP's and GDF's. Especially preferred is a combination with any one of EGF, TGF-ß1, TGF-ß2, TGF-ß3, NGF and/or GDNF.

Other acceptable components in the compositions are:
- Retinoids (vitamin A derivatives) which preserve the integrity of mucosal/ epithelial surfaces;
- Hydroxy acids (organic carboxylic acids further classified into alpha hydroxy acids (AHA) and beta hydroxyl acid (BHA)) which enhance epidermal shedding, i.e. glycolic acid, lactic acid, citric acid, mandelic acid, malic acid, and tartaric acid;
- Antioxidants which counteract the harmful effects of free radicals, i.e. vitamin C, vitamin E, panthenol, lipoic acid, ubiquinone, niacinamide, dimethylaminoethanol, spin traps, melatonin, catalase, glutathione, superoxide dismutase, peroxidase, glucpyranosides, polyphenols, cysteine, allantoin, furfuryladenine, uric acid, and carnosine;
- Depigmenting agents which alleviate hyperpigmentation, i.e. N-acetyl-4-S-cysteanimylphenol, kojic acid, arbutin, azaleic acid, paper-mulberry compound, chemical peeling agents (resorcinol, salicylic acid), Kligman's formula, Pathak's formula, and Westerhof's formula;
- Botanicals, i.e. chamomile, ginseng, Gingko biloba, curcumin, glycyrrhizin, capsaicin, and aloe vera;
- Glycosaminoglycans which support epidermal regeneration, i.e. hyaluronic acid;
- Anticellulites which mediate lipolysis, i.e. beta-adrenergic stimulators such as theobromine, theophylline, aminophylline, caffeine, epinephrine and alpha1-adrenergic stimulators such as yohimbine, piperoxane, and phentolamine;
- Enzymes such as papaine and DNA repair enzymes;
- Hormones, i.e. estrogens, progesterone, testosterone, and growth hormone;
- Antimicrobial agents, i.e. triclosan, chlorhexidine, povidone iodine, hydrogen peroxide, antidandruff preparations, zinc pyrithione;
- Chemical UV filters, i.e. 3-benzylidene camphor (3-BC) or 4-methylbenzylidene camphor (4-MBC);
- Furthermore buffers, stabilizers, preservatives, reducing agents, antioxidant chelating agents, agents that modify isotonicity, deodorants, anaesthetics, adjuvants and solubility-enhancing additives.

These are only non-limiting examples of possible additives, and a worker skilled in the art may easily add other excipients which are currently in use which are generally regarded as safe. For more information about methods for formulating a pharmaceutical composition and selection of pharmaceutically acceptable substances please see i.e. Remington's Pharmaceutical Sciences (luth ed.; Mack Publishing Company, Eaton, Pennsylvania, 1990), Wang et al. (1980), J. Parent. Drug Assn. 34 (6): 452-462 (1980); Wang et al. (1988), J. Parent. Sci. and Tech. 42: 4-26; Lachman et al. (1968), Drug and Cosmetic Industry 102(1): 36-38, 40 and 146-148; and Akers (1988)J. Parent. Sci. and Tech. 36 (5): 222-228.

The present invention further comprises the following items:

### Item 1)

A medical device composed of at least a liquid component A comprising one or more dissolved GDF-5 related proteins, and a solid component B,
characterized by
a) said liquid component A comprising 0.001 to 5% of an organic additive selected from the group consisting of trehalose, dextran sulphate, carboxymethyldextran and carboxymethyldextran sulfate, thus perpetually preventing binding of at least 75% of the contained GDF-5 related protein(s) to component B and
b) said solid component B comprising one or more amorphous thermoplastic polymers.

### Item 2)

The medical device according to the previous item, wherein said amorphous thermoplastic polymers are selected from the group consisting of polysulfone-based plastics and polyetherimides.

### Item 3)

The medical device according to any one of the previous items, wherein said solid component B comprises one or more synthetic or semi-synthetic structural elements for storage of liquid component A and/or for delivery of liquid component A to the tissue regeneration site.

### Item 4)

The medical device according to the previous item, wherein said synthetic or semi-synthetic structural element for the storage and/or delivery of liquid component A to the tissue regeneration site is selected from the group consisting of a hollow fibre, a capillary, a tubing, a tank, a container, a mesh, a spongy element, and/or a membrane.

### Item 5)

Use of a medical device according to any one of items 1 to 4 for the improved healing of wounds including diabetic and other ulcers, burns, skin injuries and/or skin grafts, for the induction of nerve growth or prevention of neuronal death, for the promotion of angiogenesis, for the induction of proliferation of progenitor cells and/or bone marrow cells; for maintenance of a state of proliferation or differentiation for treatment or preservation of tissue or cells for organ or tissue transplantation; for the treatment of degenerative disorders concerning the joints to skeletal elements and/or for meniscus and/or spinal/intervertebral disk repair.

### Item 6)

Use of a medical device according to any one of the previous claims for the promotion of tissue regeneration, said tissue being selected from the group consisting of skin tissue, connective tissue, bone, cartilage, connective tissue attachment, tendon, ligament, spinal/intervertebral disk, meniscus, dental tissue, dentin, periodontal ligament, hair, tissues of the sensory system, liver, pancreas, cardiac, blood vessel, renal, uterine and thyroid tissue, mucous membranes, endothelium, epithelium or neural tissue.

### Item 7)

A method for the prevention of binding of GDF-5 related proteins in solution to amorphous thermoplastic polymers, biodegradable materials or polyvinylpyrrolidon, characterized by adding trehalose, dextran sulfate, carboxymethyldextran or carboxymethyldextran sulfate (final concentration: 0.001 to 5 %) to said solution of GDF-5 related proteins.

### Item 8)

The method according to the previous item, wherein said biodegradable materials are selected from the group consisting of woven or nonwoven collagen, gelatine, polylactide (PLA), polyglycolide (PGA), polycaprolactone (PCL) polylactid, dextran, hyaluronic acid and chitosan or combinations thereof.

The following non-limiting examples together with the figures and sequence protocols are intended to further illustrate the invention.

SEQ ID NO:1 shows the DNA sequence, and SEQ ID NO:2 shows the protein sequence of the human GDF-5 precursor.

SEQ ID NO:3 shows the DNA sequence, and SEQ ID NO:4 shows the protein sequence of the human mature monomeric GDF-5.

### FIGURES

FIG. 1 shows additional features of the human GDF-5 precursor protein according to SEQ ID NO:2: aa 001-381 pre-prodomain (bold letters)
   aa 001-027 signal peptide (bold and underlined)
   aa 382-501 mature protein part
   aa 400-501 cystine-knot-domain (underlined)
FIG. 2 shows a comparison of the 102 aa cystine-knot domains of human GDF-5 (SEQ ID NO:2), human GDF-6 (sequence 2 from U.S. Pat. No. 5,658,882) and human GDF-7 (sequence 26 from U.S. Pat. No. 5,658,882). Amino acid residues which are identical in all three molecules are highlighted by borders.
FIG. 3 shows a table with the sequence identities of cystine-knot domains of several known BMPs and GDFs to the cysteine-knot-domain of human GDF-5.
FIG. 4 demonstrates the electrostatic charge pattern of GDF-5 dimer at neutral pH. Regions with similar charge show identical colours.
FIG. 5 shows a repeating unit of polysulfone-based plastics.
FIG. 6 shows a repeating unit of polysulfones (PSU).
FIG. 7 shows a repeating unit of polyethersulfones (PES).
FIG. 8 shows a repeating unit of polyphenylsulfones (PPSU).
FIG. 9 shows a repeating unit of polyetherimides.
FIG. 10 shows an rhGDF-5 sandwich ELISA as described in example 1. The content of rhGDF-5 dissolved in phosphate buffer saline (PBS) was compared before (black bar) and after (white bar) hollow fibre capillary passage. The rhGDF-5 content was analyzed by rhGDF-5 sandwich ELISA. After hollow fibre capillary passage no GDF-5 could be detected. Most likely the rhGDF-5 protein adhered unspecifically to the capillary walls.

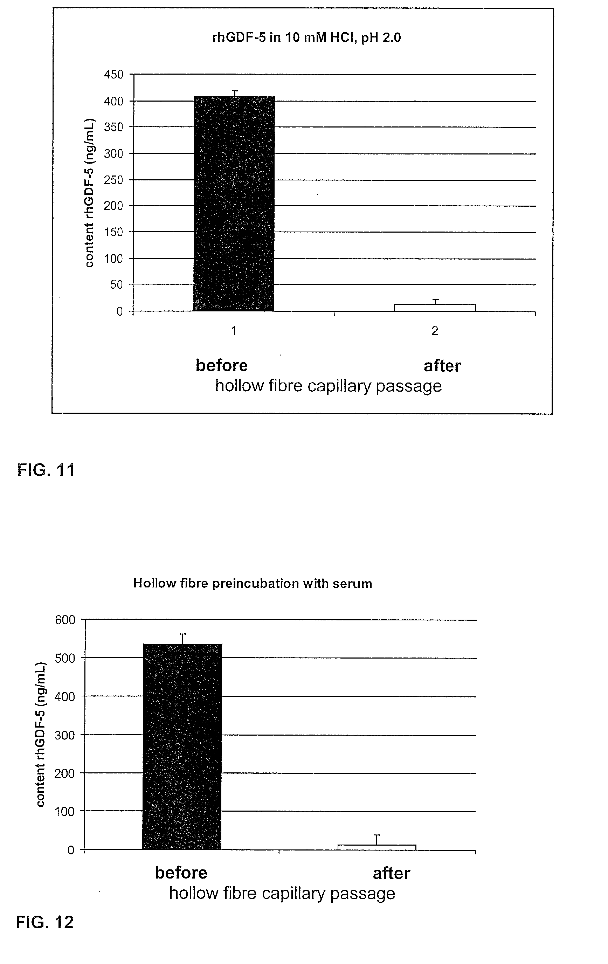

FIG.11 shows an rhGDF-5 sandwich ELISA as described in example 2. The content of rhGDF-5 dissolved in 10 mM HCl, pH 2.0 was compared before (black bar) and after (white bar) hollow fibre capillary passage. The rhGDF-5 content was analyzed by rhGDF-5 sandwich ELISA. After hollow fibre capillary passage no GDF-5 could be detected.
FIG. 12 shows an rhGDF-5 sandwich ELISA as described in example 3. Hollow fibre capillaries were preincubated with human serum to block unspecific binding sites for rhGDF-5. Then 5 mL rhGDF-5 (1 µg/mL dissolved in PBS) were injected into the device. The content of rhGDF-5 was compared before (black bar) and after (white bar) hollow fibre capillary passage. The rhGDF-5 content was analyzed by rhGDF-5 sandwich ELISA. After hollow fibre capillary passage no GDF-5 could be detected.

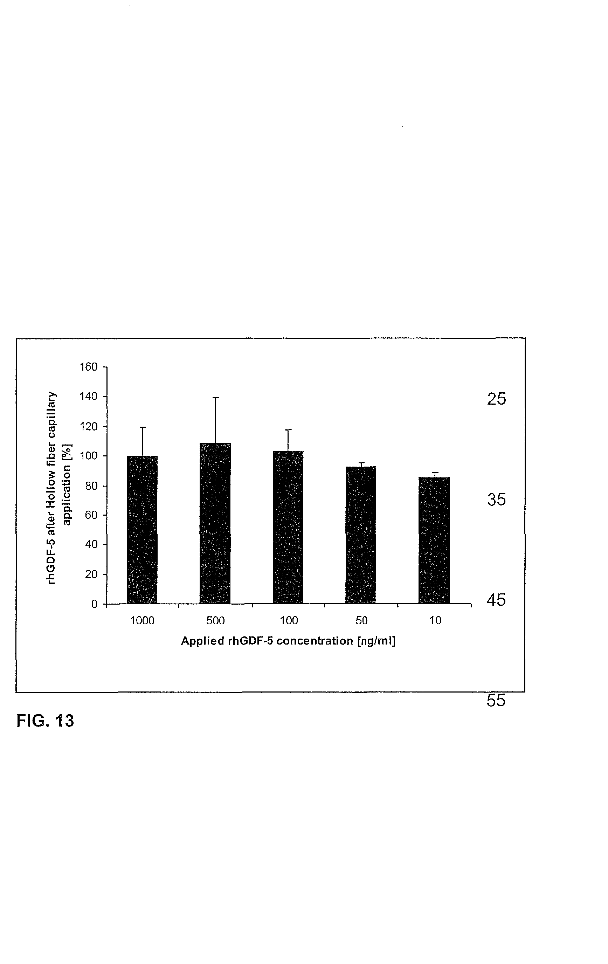

FIG. 13 shows an rhGDF-5 sandwich ELISA as described in example 4. Hollow fibre capillaries were rinsed with 1% dextran sulfate solution. Then hollow fibres were injected with 5 mL rhGDF-5 in 1% dextran sulfate with increasing rhGDF-5 concentrations ranging from 10 to 1000 ng/mL. The rhGDF-5 content before and after hollow fibre capillary injection was determined by rhGDF-5 sandwich ELISA. The rhGDF-5 input concentration was set to 100 %. rhGDF-5 could be detected in the fraction after hollow fibre capillary passage. The blocking of free binding sites with dextran sulfate did prevent the unspecific adhesion of rhGDF-5 to the capillary surface. In contrast thereto, blocking of free binding sites with serum proteins did not prevent the unspecific adhesion of rhGDF-5 to capillary surfaces.

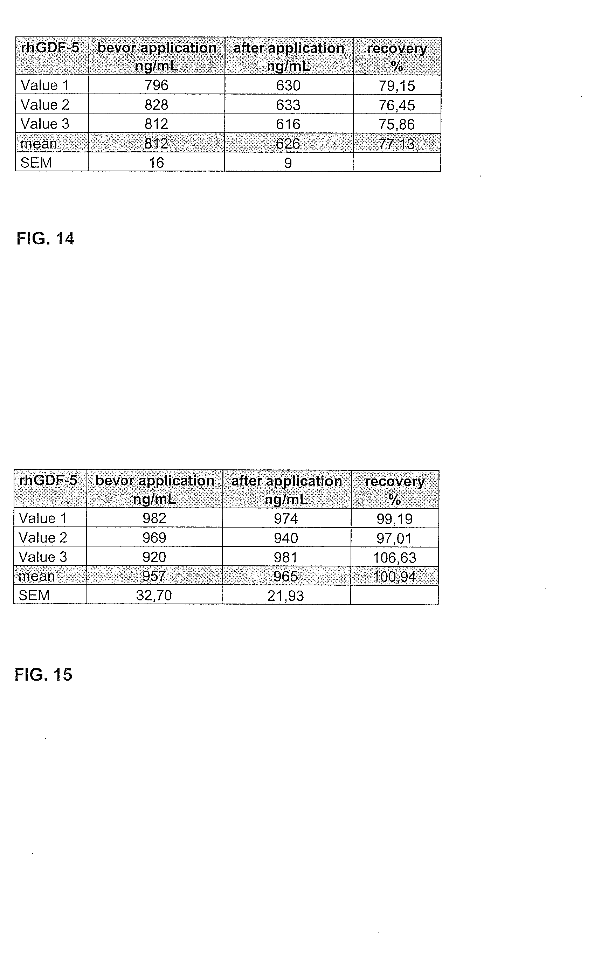

FIG. 14 shows the results of an rhGDF-5 sandwich ELISA as described in example 4. Hollow fibre capillaries were rinsed with 0.001% dextran sulfate solution. Then hollow fibres were injected with 5 mL 1 µg/mL rhGDF-5 in 0.001% dextran sulfate. The rhGDF-5 content before and after hollow fibre capillary injection was determined by rhGDF-5 sandwich ELISA.
FIG. 15 shows the results of an rhGDF-5 sandwich ELISA as described in example 5. Hollow fibre capillaries were rinsed with 1% trehalose, 5 mM sodium acetate, pH 5. Then hollow fibres were injected with 5 mL rhGDF-5 (2 µg/mL) in 1% trehalose, 5 mM sodium acetate, pH 5. The rhGDF-5 content before and after hollow fibre capillary injection was determined by rhGDF-5 sandwich ELISA.

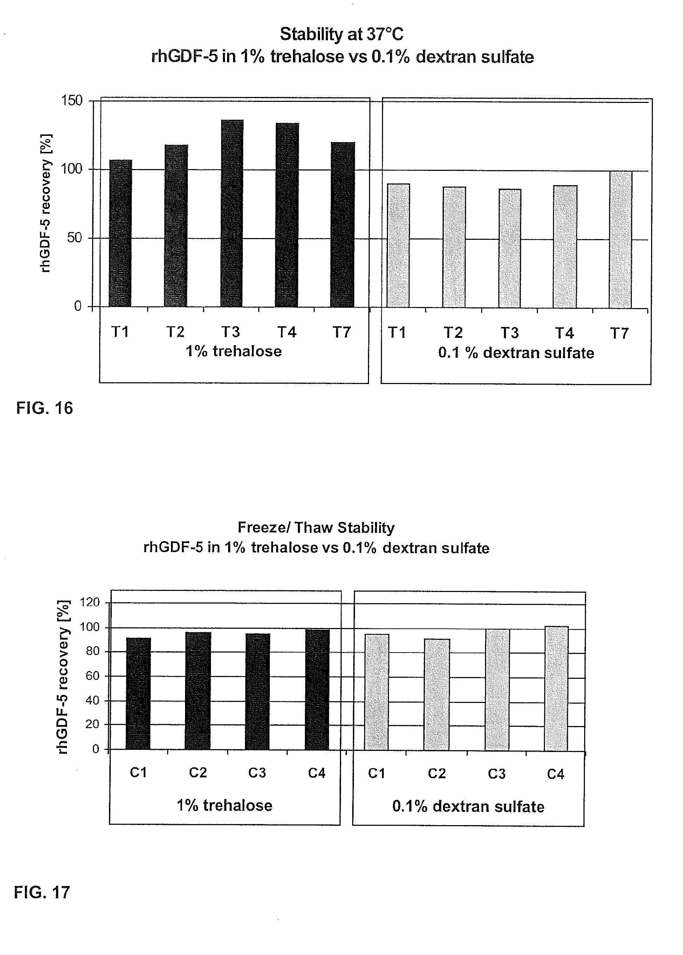

FIG 16 shows the results of a stability study for rhGDF-5 at 37°C for 1 week as described in example 6. rhGDF-5 was formulated either in 1 % trehalose or 0.1 % dextran sulfate. The rhGDF-5 content for the time point day 1 to day 7 (T1 to T7) was determined by rhGDF-5 sandwich ELISA. An untreated rhGDF-5 sample served as an internal control and was set to 100 %.
FIG. 17: shows a freeze/thaw stability study for rhGDF-5 as described in example 7. rhGDF-5 was dissolved either in 1% trehalose or 0.1% dextran sulfate. rhGDF-5 samples were frozen at -80°C and thawed at room temperature four times. The rhGDF-5 content for the freeze/thaw cycles (C1 to C4) was determined by rhGDF-5 sandwich ELISA. An untreated rhGDF-5 sample served as an internal control and was set to 100 %.

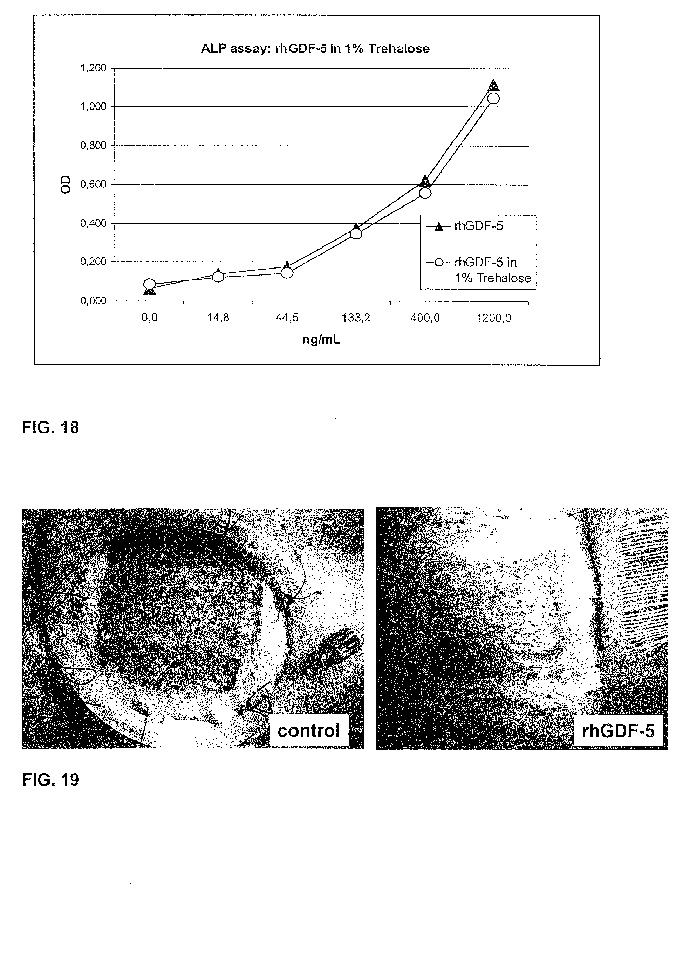

FIG. 18 shows a dose response analysis of alkaline phosphatase (ALP) activity in MCHT1/26 cells as described in example 8. MCHT1/26 cells were stimulated with 4.8-1200 ng/mL of GDF-5 dissolved in 10 mM HCl (black triangles) and in 1% trehalose, 5 mM sodium acetate pH 5.0 (white circles) respectively. ALP activity was measured by the conversion of p-nitrophenolphosphate to p-nitrophenolate at 405 nM. The data are average values of three independent measurements.
FIG. 19 shows in vivo wound healing study on pig skin according to example 9. rhGDF-5 (150 ng/mL) formulated in 1% trehalose, 5 mM sodium acetate pH 5.0, was applied via a hollow fibre application system. In contrast to the untreated control wound (left side), the rhGDF-5 treated partial-thickness defect (right side) was regenerated within 9 days.

### EXAMPLES

### Example 1: Delivery of GDF-5 related proteins in an aqueous buffer solution at neutral pH (PBS)

In a first set of experiments it was investigated if GDF-5 or related proteins in an aqueous buffer system pass a hollow fibre capillary device. As an example, rhGDF-5 was dissolved in 1x phosphate buffered saline to generate a 6 mL rhGDF-5 solution with a final concentration of 1 µg/mL. 5 mL of the GDF-5 solution were applied with a syringe to a hollow fibre capillary device. The GDF-5 solution which left the micropores of the hollow fibre capillary device was collected in a pertri dish. To quantify the content of GDF-5 which has passed the hollow fibre capillary device, an aliquot of the sample before and after capillary passage were analyzed in a GDF-5 specific sandwich ELISA. The GDF-5 ELISA is based on two monoclonal antibodies to rhGDF-5. The enzyme avidin-peroxidase is bound to the second antibody by adding a third reagent. Detection is carried out by enzymatic conversion of the substrate tetramethylbenzidine dihydrochloride into a yellow dye, which is then determined by photometry. Unknown samples of rhGDF-5 are quantified by using a test series of rhGDF-5 standards.

In brief, the first antibody aMP-5 was adjusted to 5 µg/mL in TBS (0.01 M Tris buffered saline, 0.15 M NaCl, pH 7.4) and the ELISA plate was coated with 50 µL/well and incubated overnight at 4 °C. The solution was removed and the plate was rinsed 5 times with 100 µL of rinsing solution (100 mL stock solution (0.45 M Na₂HPO₄, pH 6.5) diluted into 400 mL water p.a. and 250 µL Tween-20 was added). Next, the 50 µL/well of the standard and sample solutions were applied 4 times each, the plate was closed and incubated for 20 - 24 h at 4°C. The solutions were removed and the plate was rinsed 5 times with 100 µL rinsing solution. The second biotinylated antibody aMP-4 was adjusted to a concentration of 1 µg/mL with dilution buffer (10 mM TRIS, 150 mM NaCl, 0.5% Casein, 0.0004% bromophenol blue, pH 7.4), 50 µL/well were applied and the closed plate were incubated for 2 hour at 37 °C. The solution was removed and the plate was rinsed 5 times with 100 µL rinsing solution. 50 µL per well of avidin-peroxidase (0.2 U/mL peroxidase activity) were added and the closed plate was incubated for 1 hour at room temperature. The solution was removed and the plate was rinsed 5 times with 100 µL rinsing solution. Next, 50 µL of the TMB (tetramethylbenzidine) dye solution was added per well and the closed plate was incubated for 30 min at room temperature. Finally, the reaction was stopped by adding 50 µL 1 N H₂SO₄ per well and the absorption was measured at 450 nm.

The comparison of the rhGDF-5 content before and after passage through the hollow fibre capillary device is shown in Figure 10. Surprisingly no rhGDF-5 could be detected in the fraction after hollow fibre capillary passage. Most likely the rhGDF-5 protein adhered unspecifically to the capillary walls.

### Example 2: Delivery of GDF-5 related proteins in 10 mM HCl, pH 2.0

Since 10 mM HCl, pH 2.0, is an optimal buffer for dissolving GDF-5 related proteins, it was investigated if GDF-5 in 10 mM HCl, pH 2.0 passes a hollow fibre capillary device. The procedure was performed under the same (1µg/mL) was applied in 10 mM HCl, pH 2 instead of PBS.

The comparison of the rhGDF-5 content, dissolved in 10 mM HCL, pH 2.0, before and after passage through the hollow fibre capillary device is shown in FIG 11.

Surprisingly no rhGDF-5 could be detected in the fraction after hollow fibre capillary passage. Since 10 mM HCl, pH 2.0 is an optimal buffer for rhGDF-5 to achieve maximal protein solubility; it is obvious that good protein solubility is not sufficient to prevent adhesion to the capillary material. Therefore alternative strategies to reduce unspecific rhGDF-5 interaction were applied.

### Reference Example 3: Prevention of unspecific binding by blocking with serum proteins

One strategy to reduce unwanted protein interaction is to block the free binding sites of the capillaries with high amounts of proteins (e.g. human serum albumin). For the blocking procedure, the hollow fibre capillary device was preincubated with 5 mL human serum. After the blocking step, 5 mL rhGDF-5 (1µg/mL) dissolved in PBS were injected into the hollow fibre capillary device. The rhGDF-5 content before and after passage through the hollow fibre capillary device was analyzed as described in Example 1. The results are shown in Figure 12.

rhGDF-5 could be not detected in the fraction after hollow fibre capillary passage. The blocking of free binding sites with serum proteins did not prevent the unspecific adhesion of rhGDF-5 to the capillary surface.

### Example 4: Prevention of unspecific rhGDF-5 binding by addition of dextran sulfate

Another strategy to reduce unwanted protein interaction is to add additives to the rhGDF-5 solution (e.g. dextran sulfate or trehalose). For this purpose rhGDF-5 was dissolved in a buffer comprising 1% (10 mg/ml) dextran sulfate (Amersham 17-0340-01, Lot 99250) in water. In this experiment the following rhGDF-5 concentrations were analyzed, 10 ng/mL, 50, ng/mL, 100 ng/mL, 500 ng/mL and 1000 ng/mL.

The hollow fibre capillary device was first rinsed with 5 mL 1 % dextran sulfate solution without rhGDF-5. After the rinsing step 5 mL rhGDF-5 in 1% dextran sulfate solution (rhGDF-5 concentrations 10 to 1000 ng/mL) was injected separately into the hollow fibre capillary device. The rhGDF-5 content of all 5 samples before and after passage through the hollow fibre capillary device were analyzed by rhGDF-5 ELISA as described in Example 1. The results are shown in Figure 13.

The addition of 1% dextran sulfate to rhGDF-5 a solution prevents successful the unspecific binding of rhGDF-5 to the hollow fibre capillaries. In an rhGDF-5 concentration range of 1000 to 100 ng/mL, 100 % of the injected rhGDF-5 could be detected after hollow fibre capillary passage. The rhGDF-5 recovery for 50 ng/mL rhGDF-5 was 92 % and for 10 ng/mL 85 % respectively.

The experiments with dextran sulfate were repeated with a reduced dextran sulfate concentration (0.001 % instead of 1%) and 1 µg/ mL rhGDF-5. Even with a dextran sulfate concentration of 0.001 % the rhGDF-5 recovery after hollow fibre capillary passage was greater than 75 % (FIG. 14).

### Example 5: Prevention of unspecific rhGDF-5 binding by addition of trehalose

An alternative carbohydrate additive to dextran sulfate is trehalose. The experiments with trehalose were conducted as described in Example 4, with the exception that trehalose was used instead of dextran sulfate.

2 µg/mL rhGDF-5 were dissolved either in 1% or 10% trehalose, 5 mM sodium acetate pH 5.0. The hollow fibre capillary device was first rinsed with 5 mL 1% or 10% trehalose, 5 mM sodium acetate solution without rhGDF-5. After the rinsing step 5 mL rhGDF-5 (2 µg/mL) in 1% or 10% trehalose, 5 mM sodium acetate solution were injected into the hollow fibre capillary device. The rhGDF-5 content before and after passage through the hollow fibre capillary device was analyzed by rhGDF-5 ELISA as described in Example 1.

The results are shown in FIG. 15. Surprisingly no rhGDF-5 could be detected in the fraction after hollow fibre capillary passage when the rhGDF-5 solution with 10% trehalose was used. In contrast, when rhGDF-5 was dissolved in 1% trehalose, 5 mM sodium acetate, pH 5, the rhGDF-5 recovery after hollow fibre capillary passage was 100%.

### Example 6: rhGDF-5 temperature stability study with trehalose and dextran sulfate

An important criteria for a wound healing drug formulation is the drug stability at body temperature. Therefore a short term stability study for rhGDF-5 in the presence of either 1 % trehalose or 0.1 % dextran sulfate was performed for a period of one week at 37°C. For this purpose 6 aliquots with 30 µl were prepared, containing 200 µg/mL rhGDF-5 dissolved in 1% trehalose, 5 mM sodium acetate pH 5.0 and 0.1% dextran sulfate in water respectively. The rhGDF-5 solutions were stored at 37°C for 1 week; one aliquot was removed each day and stored at -80°C until the stability study was finished at day 7. The rhGDF-5 content of the temperature stressed samples was compared against an rhGDF-5 aliquot which was directly stored at - 80°C without undergoing a stress test. The samples were analyzed by rhGDF-5 sandwich ELISA described under Example 1. The results are shown in Figure 16. It could be demonstrated that rhGDF-5 is stable at 37°C at least for one week, when formulated in 1% trehalose, 5 mM sodium acetate pH 5.0 or 0.1% dextran sulfate in aqueous buffers.

### Example 7: rhGDF-5 freeze/thaw stability study with trehalose and dextran sulfate

Another important aspect for drug stability is that the additives in the drug formulation supports repeated freeze/thaw cycles of rhGDF-5 without loosing bioactivity. The stability study for rhGDF-5 in the presence of either 1% trehalose or 0.1 % dextran sulfate was performed for a period of one week at 37°C. For this purpose aliquots with 30 µl were prepared, containing 200 µg/mL rhGDF-5 dissolved in 1% trehalose, 5 mM sodium acetate pH 5.0 and 0.1% dextran sulfate in 1x phosphate buffered saline respectively. The rhGDF-5 solutions were stored at - 80°C over night and then thawed at room temperature. The freeze/thaw cycle was repeated four times (C1 to C4). The rhGDF-5 content of the stressed samples was compared against an rhGDF-5 aliquot which was directly stored at - 80°C without undergoing a stress test. The samples were analyzed by rhGDF-5 sandwich ELISA described under Example 1. The results are shown in Figure 17. It could be demonstrated that rhGDF-5 could be frozen and thawed at least four times without loosing activity, when formulated in 1% trehalose, 5 mM sodium acetate pH 5.0 or 0.1 % dextran sulfate in aqueous buffers.

### Example 8: Bioactivity study with rhGDF-5 in 1% trehalose

To address the question if the carbohydrate additives in the rhGDF-5 formulation buffer have an influence on the GDF-5 bioactivity, a cell based alkaline phosphatase (ALP) activity assay was performed. The biological activity of rhGDF-5 (200 µg/mL) dissolved in 1% trehalose, 5 mM sodium acetate pH 5.0 was measured on mouse stromal MCHT1/26 cells (Hoechst Japan Ltd., Kawagoe, Japan). MCHT1/26 cells were plated at 4.5 x 10³ cells per well in 96-multiwell plates in cell culture medium (alpha-MEM, (Sigma, Taufkirchen, Germany) supplemented by 2 mM L-glutamine, (Invitrogen, Karlsruhe, Germany) and 10% fetal calf serum (Invitrogen, Karlsruhe, Germany). After 24 h, cells were stimulated with rhGDF-5 dissolved either in 10 mM HCl or in 1% trehalose, 5 mM sodium acetate pH 5.0. Prior to cell incubation, the rhGDF-5 solutions were further diluted with cell culture medium to reach rhGDF-5 concentrations ranging from 14.8 - 1200 ng/mL. After 72 h, cells were washed with phosphate buffered saline (PBS) and extracted with alkaline phosphate buffer 1, containing 1% Nonidet P40, 0.1 M glycine pH 9.6 (Sigma, Taufkirchen, Germany), 1 mM MgCl₂ and 1 mM ZnCl₂ (Merck, Darmstadt, Germany). To achieve thorough cell lysis, cells were incubated 15 - 18 h at 37°C. Alkaline phosphatase enzyme activity was assayed with 10 mM p-nitrophenylphosphate (Pierce, Bonn, Germany) as a substrate in 0.1 M glycine pH 9.6, 1 mM MgCl₂ and 1 mM ZnCl₂. After 30 min incubation at 37°C, the absorbance was measured with an automatic microplate reader (Tecan Spectra Rainbow, TECAN, Crailsheim, Germany) at 405 nM under consideration of blank value subtraction. The results are shown in Figure 18.

rhGDF-5 was tested for its biological activity to induce ALP enzyme activation in the indicator cell line MCTH1/26 in the presence of 1% trehalose. Compared to the rhGDF-5 control sample, no influence of trehalose on the rhGDF-5 induced ALP activity could be observed. The induction of ALP activity by rhGDF-5 in the presence of 1% trehalose was dose dependently, suggesting that trehalose does not reduce the biological activity of GDF-5.

### Example 9: Wound healing in vivo study with rhGDF-5 in 1% trehalose applied via hollow fibre capillary device

Since wound healing is a severe problem in patients suffering from diabetes and in patients with severe burns, we have developed a dermal delivery device for rhGDF-5 to foster the healing process of chronic wounds which do not heal by themselves.

The delivery system consists of a hollow fibre network which is connected to inlet and outlet tubing, allowing the perfusion with an rhGDF-5 enriched medium. We have successfully developed a drug formulation for GDF-5, preventing the adhesion of GDF-5 to the surface of the hollow fibre walls. Since GDF-5 is a sticky protein with an unusual solubility profile, the formulation is essential for an effective rhGDF-5 delivery.

GDF-5 has a low solubility in aqueous solution at neutral pH (approx. 1 µg/mL), it is soluble within the pH ranges 2.0 to pH 4.5 and between pH 9.5 to 12.0. To reach good solubility for GDF-5, the protein should be dissolved in 10 mM HCl, pH 2.0 or in 20 mM NaAcetate buffer, pH 4.0.
To investigate the influence of rhGDF-5 on cell growth, cell culture assays on isolated human cells were performed. Fibroblasts and keratinocytes were directly isolated from human skin biopsies and incubated with rhGDF-5. Interestingly, a very low concentration of rhGDF-5 (75 ng/mL) leads to an increase of cell growth of about 30%. We suggest that this effect accelerates the wound healing and regeneration process of chronic wounds (e.g. diabetic foot ulcer).

Subsequently wound healing experiments in animals with rhGDF-5 were performed. Partial thickness skin wounds (5x5 cm) were set on a pig back. Wounds were treated with rhGDF-5 (150 ng/mL rhGDF-5, 1% trehalose, 5 mM sodium acetate pH 5.0.) using a hollow fibre capillary application system. 10 mL rhGDF-5 solution were applied twice daily via the hollow fibre capillary device over a period of 9 days. The rhGDF-5 treated wounds regenerated significantly faster (Figure 19).

## Claims

1. A medical device composed of at least a liquid component A comprising one or more dissolved GDF-5 related proteins, and a solid component B, **characterized by**
a) said liquid component A comprising 0.001 to 5% of an organic additive selected from the group consisting of trehalose, dextran sulphate, carboxymethyldextran and carboxymethyldextran sulfate, thus perpetually preventing binding of at least 75% of the contained GDF-5 related protein(s) to component B and
b) said solid component B comprising one or more amorphous thermoplastic polymers,
wherein the GDF-5 related protein is a protein which comprises a cystine-knot domain with an amino acid identity of at least 60% to the 102 aa cystine-knot domain of human GDF-5 (amino acids 400-501 of SEQ ID NO: 2).

2. The medical device according to the previous claim, wherein said amorphous thermoplastic polymers are selected from the group consisting of polysulfone-based plastics and polyetherimides.

3. The medical device according to any one of the previous claims, wherein said solid component B comprises one or more synthetic or semi-synthetic structural elements for storage of liquid component A and/or for delivery of liquid component A to the tissue regeneration site.

4. The medical device according to the previous claim, wherein said synthetic or semi-synthetic structural element for the storage and/or delivery of liquid component A to the tissue regeneration site is selected from the group consisting of a hollow fibre, a capillary, a tubing, a tank, a container, a mesh, a spongy element, and/or a membrane.

5. Medical device according to any one of claims 1 to 4 for use in the improved healing of wounds including diabetic and other ulcers, burns, skin injuries and/or skin grafts, the induction of nerve growth or prevention of neuronal death, the promotion of angiogenesis, the induction of proliferation of progenitor cells and/or bone marrow cells; maintenance of a state of proliferation or differentiation for treatment or preservation of tissue or cells for organ or tissue transplantation; the treatment of degenerative disorders concerning the joints to skeletal elements and/or meniscus and/or spinal/intervertebral disk repair.

6. Medical device according to any one of the previous claims for use in the promotion of tissue regeneration, said tissue being selected from the group consisting of skin tissue, connective tissue, bone, cartilage, connective tissue attachment, tendon, ligament, spinal/intervertebral disk, meniscus, dental tissue, dentin, periodontal ligament, hair, tissues of the sensory system, liver, pancreas, cardiac, blood vessel, renal, uterine and thyroid tissue, mucous membranes, endothelium, epithelium or neural tissue.

7. A method for the prevention of binding of GDF-5 related proteins in solution to amorphous thermoplastic polymers, biodegradable materials or polyvinylpyrrolidon, **characterized by** adding trehalose, dextran sulfate, carboxymethyldextran or carboxymethyldextran sulfate (final concentration: 0.001 to 5 %) to said solution of GDF-5 related proteins, wherein the GDF-5 related protein is a protein which comprises a cystine-knot domain with an amino acid identity of at least 60% to the 102 aa cystine-knot domain of human GDF-5 (amino acids 400-501 of SEQ ID NO: 2).

8. The method according to the previous claim, wherein said biodegradable materials are selected from the group consisting of woven or nonwoven collagen, gelatine, polylactide (PLA), polyglycolide (PGA), polycaprolactone (PCL) polylactid, dextran, hyaluronic acid and chitosan or combinations thereof.

## Patentansprüche

1. Medizinische Vorrichtung, gebildet aus zumindest einer flüssigen Komponente A, umfassend ein oder mehrere gelöste GDF-5-verwandte Proteine, und einer festen Komponente B, **dadurch gekennzeichnet, dass**
a) die flüssige Komponente A 0,001 bis 5% eines organischen Additivs, ausgewählt aus der Gruppe, bestehend aus Trehalose, Dextransulfat, Carboxymethyldextran und Carboxymethyldextransulfat, umfasst, somit dauerhaft Bindung von zumindest 75% des/der enthaltenen GDF-5-verwandten Protein(e) an Komponente B verhindert, und
b) die feste Komponente B ein oder mehrere amorphe thermoplastische Polymere umfasst,
wobei das GDF-5-verwandte Protein ein Protein ist, welches eine Cystin-Knoten-Domäne mit einer Aminosäureidentität von zumindest 60% zu der 102 aa Cystin-Knoten-Domäne von humanem GDF-5 (Aminosäuren 400-501 von SEQ ID NO:2) umfasst.

2. Medizinische Vorrichtung nach dem vorangehenden Anspruch, wobei die amorphen thermoplastischen Polymere ausgewählt sind aus der Gruppe, bestehend aus Polysulfon-basierten Kunststoffen und Polyetherimiden.

3. Medizinische Vorrichtung nach einem der vorangehenden Ansprüche, wobei die feste Komponente B ein oder mehrere synthetische oder semi-synthetische strukturelle Elemente zur Lagerung der flüssigen Komponente A und/oder zur Bereitstellung der flüssigen Komponente A an der Geweberegenerierungsstelle umfasst.

4. Medizinische Vorrichtung nach dem vorangehenden Anspruch, wobei das synthetische oder semi-synthetische strukturelle Element zur Lagerung und/oder zur Bereitstellung der flüssigen Komponente A an der Geweberegenerierungsstelle ausgewählt ist aus der Gruppe, bestehend aus einer Hohlfaser, einer Kapillare, einem Schlauch, einem Tank, einem Behälter, einem Netz, einem schwammartigen Element und/oder einer Membran.

5. Medizinische Vorrichtung nach einem der Ansprüche 1 bis 4 zur Verwendung bei verbesserter Wundheilung, umfassend diabetische und andere Geschwüre, Verbrennungen, Hautverletzungen und/oder Hauttransplantate, der Induzierung von Nervenwachstum oder Verhinderung von Absterben von Neuronen, der Förderung von Angiogenese, der Induzierung der Proliferation von Vorläuferzellen und/oder Knochenmarkzellen; Aufrechterhaltung eines Zustands der Proliferation oder Differenzierung zur Behandlung oder Erhaltung von Gewebe oder Zellen für Organ- oder Gewebetransplantation; der Behandlung von degenerativen Erkrankungen betreffend die Gelenke skelettaler Elemente und/oder Meniskus- und/oder Band-/Zwischenwirbelscheiben-Reparatur.

6. Medizinische Vorrichtung nach einem der vorangehenden Ansprüche zur Verwendung in der Förderung von Geweberegenerierung, wobei das Gewebe ausgewählt ist aus der Gruppe, bestehend aus Hautgewebe, Bindegewebe, Knochen-, Knorpel-, Bindegewebebefestigung, Sehne, Band, Band-/Zwischenwirbelscheibe, Meniskus, Dentalgewebe, Zahnbein, Wurzelhaut, Haar, Geweben des sensorischen Systems, Leber-, Pankreas-, Herz-, Blutgefäß, Nieren-, Uterus- und Schilddrüsengewebe, Schleimhäuten, Endothel, Epithel oder Nervengewebe.

7. Verfahren zur Verhinderung der Bindung von GDF-5-verwandten Proteinen in Lösung an amorphe thermoplastische Polymere, biologisch abbaubare Materialien oder Polyvinylpyrrolidon, **dadurch gekennzeichnet, dass** Trehalose, Dextransulfat, Carboxymethyldextran oder Carboxymethyldextransulfat (Endkonzentration: 0,001 bis 5 %) zu der Lösung GDF-5-verwandter Proteine zugegeben wird, wobei das GDF-5-verwandte Protein ein Protein ist, welches eine Cystin-Knoten-Domäne mit einer Aminosäureidentität von zumindest 60% zu der 102 aa Cystin-Knoten-Domäne von humanem GDF-5 (Aminosäuren 400-501 von SEQ ID NO: 2) umfasst.

8. Verfahren nach dem vorangehenden Anspruch, wobei die biologisch abbaubaren Materialien ausgewählt sind aus der Gruppe, bestehend aus gewebtem oder nichtgewebtem Kollagen, Gelatine, Polylactid (PLA), Polyglycolid (PGA), Polycaprolacton (PCL) Polylactid, Dextran, Hyaluronsäure und Chitosan oder Kombinationen davon.

## Revendications

1. Matériel médical composé d'au moins un composant liquide A comprenant une ou plusieurs protéines apparentées au GDF-5 dissoutes, et un composant solide B, **caractérisé par**
a) ledit composant liquide A comprenant 0,001 à 5% d'un additif organique choisi dans le groupe constitué par le tréhalose, le sulfate de dextran, le carboxyméthyldextran et le sulfate de carboxyméthyldextran, empêchant ainsi perpétuellement la liaison d'au moins 75% de la (ou des) protéine (s) apparentée(s) à GDF-5 contenue(s) au composant B et
b) ledit composant solide B comprenant un ou plusieurs polymères thermoplastiques amorphes,
dans lequel la protéine apparentée à GDF-5 est une protéine qui comprend un domaine à noeud de cystine avec une identité d'acides aminés d'au moins 60% au domaine à noeud de cystine de 102 aa du GDF-5 humain (acides aminés 400 à 501 de SEQ ID No: 2).

2. Matériel médical selon la revendication précédente, dans lequel lesdits polymères thermoplastiques amorphes sont choisis dans le groupe constitué par des plastiques à base de poly(sulfone) et des poly(étherimides).

3. Matériel médical selon l'une quelconque des revendications précédentes, dans lequel ledit composant solide B comprend un ou plusieurs éléments structurels synthétiques ou semi-synthétiques pour le stockage du composant liquide A et/ou l'administration du composant liquide A au site de régénération tissulaire.

4. Matériel médical selon la revendication précédente, dans lequel ledit élément structurel synthétique ou semi-synthétique pour le stockage et/ou d'administration de composant liquide A au site de régénération tissulaire est choisi dans le groupe constitué par une fibre creuse, un capillaire, une tubulure, un réservoir, un récipient, un filet, un élément spongieux, et/ou une membrane.

5. Matériel médical selon l'une quelconque des revendications 1 à 4 pour utilisation dans la cicatrisation améliorée de plaies y compris les ulcères diabétiques et autres, les brûlures, les lésions cutanées et/ou les greffes de peau, l'induction de croissance nerveuse ou la prévention de la mort neuronale, la promotion de l'angiogenèse, l'induction de la prolifération de cellules progénitrices et/ou de cellules de la moelle osseuse ; le maintien d'un état de prolifération ou de différentiation pour le traitement ou la préservation de tissu ou de cellules pour une transplantation d'organe ou de tissu ; le traitement de troubles dégénératifs concernant les articulations des éléments du squelette et/ou la réparation de ménisque et/ou de disque spinal/intervertébral.

6. Matériel médical selon l'une quelconque des revendications précédentes pour utilisation dans la promotion de la régénération tissulaire, ledit tissu étant choisi dans le groupe constitué par du tissu cutané, du tissu conjonctif, de l'os, du cartilage, une attache du tissu conjonctif, un tendon, un ligament, un disque spinal/intervertébral, un ménisque, du tissu dentaire, de la dentine, un ligament parodontal, des cheveux, des tissus du système sensoriel, du tissu de foie, de pancréas, cardiaque, de vaisseau sanguin, rénal, utérin et de la thyroïde, des membranes muqueuses, de l'endothélium, de l'épithélium ou du tissu nerveux.

7. Procédé de prévention de liaison de protéines apparentées au GDF-5 en solution à des polymères thermoplastiques amorphes, des matériaux biodégradables ou la poly(vinylpyrrolidone), **caractérisé par** l'ajout de tréhalose, de sulfate de dextran, de carboxyméthyldextran ou de sulfate de carboxyméthyldextran (concentration finale : 0,001 à 5%) à ladite solution de protéines apparentées au GDF-5, dans lequel la protéine apparentée au GDF-5 est une protéine qui comprend un domaine à noeud de cystine avec une identité d'acides aminés d'au moins 60% au domaine de noeud de cystine de 102 aa du GDF-5 humain (acides aminés 400 à 501 de SEQ ID NO: 2).

8. Procédé selon la revendication précédente, dans lequel lesdits matériaux biodégradables sont choisis dans le groupe constitué par le collagène tissé ou non tissé, la gélatine, le poly(lactide) (PLA), le poly(glycolide) (PGA), le poly(caprolactone) (PCL) poly(lactide), le dextran, l'acide hyaluronique et le chitosan ou leurs combinaisons.
